# EUROPEAN PATENT SPECIFICATION

(11) **EP 1 815 016 B1**
(45) Date of publication and mention of the grant of the patent: **27.06.2012**
(21) Application number: 05824553.1
(22) Date of filing: 01.11.2005
(51) Int. Cl.: C12Q 1/68

(54) **COMPOSITIONS AND METHODS FOR DIAGNOSING COLON DISORDERS**
ZUSAMMENSETZUNGEN UND VERFAHREN ZUR DIAGNOSE VON KOLONERKRANKUNGEN
COMPOSITIONS ET METHODES POUR LE DIAGNOSTIC DE TROUBLES DU COLON

(30) Priority: 01.11.2004 US 623771 P; 26.01.2005 US 646592 P
(43) Date of publication of application: 08.08.2007
(60) Divisional application: 10180021.7
(73) Proprietor: George Mason University, Fairfax, VA 22030 (US); Rush University, Chicago, IL 60612 (US)
(72) Inventor: GILLEVET, Patrick, c/o George Mason University, Manassas, VA 20110 (US)
(74) Representative: Vossius & Partner
(86) International application number: PCT/US2005/039887
(87) International publication number: WO 2006/050479

(56) References cited:
- EP-A- 1 215 285
- WO-A-01/36683
- WO-A-02/08459
- WO-A-02/27014
- WO-A-99/45955
- WO-A-02/072883
- WO-A-03/053220
- OTT S J ET AL: "Reduction in diversity of the colonic mucosa associated bacterial microflora in patients with active inflammatory bowel disease." GUT. MAY 2004, vol. 53, no. 5, May 2004 (2004-05), pages 685-693, XP002384046 ISSN: 0017-5749
- SEKSIK P ET AL: "Alterations of the dominant faecal bacterial groups in patients with Crohn's disease of the colon." GUT. FEB 2003, vol. 52, no. 2, February 2003 (2003-02), pages 237-242, XP002384047 ISSN: 0017-5749
- LINSKENS, HUIJSDENS, SAVELKOUL, ET AL: "The Bacterial Flora in inflammatry Bowel Disease: Current Insights in Pathogenesis and the Influence of Antibiotics and Probiotics" SCAN J GASTROENTEROL, vol. 36, no. S234, 2001, pages 29-40, XP008064939
- KLEESSEN, KROESEN, BUHR AND BLAUT: "Mucosal and Invading Bacteria in Patients with Inflammatory Bowel Disease Compared with Controls" SCAND J GASTROENTEROL, vol. 37, 2002, pages 1034-1041, XP008065022
- SWIDSINSKI, LADHOFF, PERNTHALER, SWIDSINSKI, LOENIG-BAUCKE, ORTNER, WEBER, HOFFMANN, SCHREIBER, DIETEL, AND LOCHS: "Mucosal Flora in INflammatory Bowel Disease" GASTROENTEROLOGY, vol. 122, 2002, pages 44-54, XP002384139
- ROGERS G B ET AL: "Bacterial diversity in cases of lung infection in cystic fibrosis patients: 16S ribosomal DNA (rDNA) length heterogeneity PCR and 16S rDNA terminal restriction fragment length polymorphism profiling." JOURNAL OF CLINICAL MICROBIOLOGY. AUG 2003, vol. 41, no. 8, August 2003 (2003-08), pages 3548-3558, XP002400355 ISSN: 0095-1137
- SOKOL HARRY ET AL: "Specificities of the fecal microbiota in inflammatory bowel disease." INFLAMMATORY BOWEL DISEASES. FEB 2006, vol. 12, no. 2, February 2006 (2006-02), pages 106-111, XP008064925 ISSN: 1078-0998
- SWIDSINSKI ALEXANDER ET AL: "Spatial organization and composition of the mucosal flora in patients with inflammatory bowel disease." JOURNAL OF CLINICAL MICROBIOLOGY. JUL 2005, vol. 43, no. 7, July 2005 (2005-07), pages 3380-3389, XP002384140 ISSN: 0095-1137
- SMITH, COFFEY, KELL, O'SULLIVAN, REDMOND AND KIRWAN: "A characterization of anaerobic colonization and associated mucosal adaptations in the undiseased ileal pouch" COLORECTAL DISEASE, vol. 7, 2005, pages 563-570, XP002384141
- IWAYA, IIAI, OKAMOTO, AJIOKA, ET AL: "Change in the bacterial flora of Pouchitis" HEPATO-GASTROENTEROLOGY, vol. 53, 2006, pages 55-59, XP008065029

### SEQUENCE LISTING

<110> GEORGE MASON UNIVERSITY
<120> COMPOSITIONS AND METHODS FOR DIAGNOSING COLON DISORDERS
<130> GMU-001-WO
<140>
   <141>
<150> 60/646,592
   <151> 2005-01-26
<150> 60/673,771
   <151> 2004-11-01
<160> 38
<170> PatentIn version 3.3
<210> 1
   <211> 1489
   <212> RNA
   <213> Bacteroides distasonis
<220>
   <221> modified_base
   <222> (39)..(39)
   <223> n is a, c, g, or u
<220>
   <221> modified_base
   <222> (99) .. (99)
   <223> n is a, c, g, or u
<220>
   <221> modified_base
   <222> (101)..(101)
   <223> n is a, c, g, or u
<220>
   <221> modified_base
   <222> (108)..(108)
   <223> n is a, c, g, or u
<220>
   <221> modified_base
   <222> (235)..(235)
   <223> n is a, c, g, or u
<220>
   <221> modified_base
   <222> (482)..(482)
   <223> n is a, c, g, or u
<220>
   <221> modified_base
   <222> (656)..(656)
   <223> n is a, c, g, or u
<220>
   <221> modified_base
   <222> (932)..(932)
   <223> n is a, c, g, or u
<220>
   <221> modified_base
   <222> (936)..(936)
   <223> n is a, c, g, or u
<220>
   <221> modified_base
   <222> (1160)..(1160)
   <223> n is a, c, g, or u
<400> 1
   caauuuaaac aacgaagagu uugauccugg cucaggauna acgcuagcga caggcuuaac      60
   acaugcaagu cgaggggcac gcgcgrguag caauaccgng ngcuggcnac cggcgcacgg     120
   gugaguaacg cguaugcaac uugccuauca gagggggaua acccggcgaa agucggacua     180
   auaccgcaug aagcagggau cccgcauggg aauauuugcu aaagauucau cgcunauaga     240
   uaggcaugcg uuccauuagg caguuggcgg gguaacggcc caccaaaccg acgauggaua     300
   gggguucuga gaggaagguc cccacauugg guacugagac acggaccaaa cuccuacggg     360
   aggcagcagu gaggaauauu ggucaauggc cgagaggcug aaccagccaa gucgcgugag     420
   ggaugaaggu ucuauggauc guaaaccucu uuuauaaggg aauaaagugc gggacguguc     480
   cnguuuugua uguaccuuau gaauaaggau cggcuaacuc cgugccagca gccgcgguaa     540
   uacggaggau ccgagcguua uccggauuua uuggguuuaa agggugcgua ggcggccuuu     600
   uaagucagcg gugaaagucu guggcucaac cauagaauug ccguugaaac uggggngcuu     660
   gaguauguuu gaggcaggcg gaaugcgugg uguagcggug aaaugcauag auaucacgca     720
   gaaccccgau ugcgaaggca gccugccaag ccauuacuga cgcugaugca cgaaagcgug     780
   gggaucaaac aggauuagau acccugguag uccacgcagu aaacgaugau cacuagcugu     840
   uugcgauaca cuguaagcgg cacagcgaaa gcguuaagug auccaccugg ggaguacgcc     900
   ggcaacggug aaacucaaag gaauugacgg gngccngcac aagcggagga acaugugguu     960
   uaauucgaug auacgcgagg aaccuuaccc ggguuugaac gcauucggac cgagguggaa    1020
   acaccuuuuc uagcaauagc cguuugcgag gugcugcaug guugucguca gcucgugccg    1080
   ugaggugucg gcuuaagugc cauaacgagc gcaacccuug ccacuaguua cuaacagguu    1140
   aggcugagga cucugguggn acugccagcg uaagcugcga ggaaggcggg gaugacguca 1200
   aaucagcacg gcccuuacau ccggggcgac acacguguua caauggcgug gacaaaggga 1260
   ggccaccugg cgacagggag cgaaucccca aaccacgucu caguucggau cggagucugc 1320
   aacccgacuc cgugaagcug gauucgcuag uaaucgcgca ucagccaugg cgcggugaau 1380
   acguucccgg gccuuguaca caccgcccgu caagccaugg gagccggggg uaccugaagu 1440
   ccguaaccga aaggaucggc cuagguaaaa acuggugacu ggggcuaag 1489
<210> 2
   <211> 1533
   <212> RNA
   <213> Bacteroides fragilis
<400> 2
   uuacaacgaa gaguuugauc cuggcucagg augaacgcua gcuacaggcu uaacacaugc 60
   aagucgaggg gcaucaggaa gaaagcuugc uuucuuugcu ggcgaccggc gcacggguga 120
   guaacacgua uccaaccugc ccuuuacucg gggauagccu uucgaaagaa agauuaauac 180
   ccgauagcau aaugauuccg caugguuuca uuauuaaagg auuccgguaa aggaugggga 240
   ugcguuccau uagguuguug gugagguaac ggcucaccaa gccuucgaug gauagggguu 300
   cugagaggaa ggucccccac auuggaacug agacacgguc caaacuccua cgggaggcag 360
   cagugaggaa uauuggucaa ugggcgcuag ccugaaccag ccaaguagcg ugaaggauga 420
   aggcucuaug ggucguaaac uucuuuuaua uaagaauaaa gugcaguaug uauacuguuu 480
   uguauguauu auaugaauaa ggaucggcua acuccgugcc agcagccgcg guaauacgga 540
   ggauccgagc guuauccgga uuuauugggu uuaaagggag cguaggugga cugguaaguc 600
   aguugugaaa guuugcggcu caaccguaaa auugcagcug auacugucag ucuugaguac 660
   aguagaggug ggcggaauuc gugguguagc ggugaaaugc uuagauauca cgaagaacuc 720
   cgauugcgaa ggcagcucac uggacugcaa cugacacuga ugcucgaaag uguggguauc 780
   aaacaggauu agauacccug guaguccaca caguaaacga ugaauacucg cuguuugcga 840
   uauacaguaa gcggccaagc gaaagcauua aguauuccac cuggggagua cgccggcaac 900
   ggugaaacuc aaaggaauug acgggggccc gcacaagcgg aggaacaugu gguuuaauuc 960
   gaugauacgc gaggaaccuu acccgggcuu aaauugcagu ggaaugaugu ggaaacaugu 1020
   cagugagcaa ucaccgcugu gaaggugcug caugguuguc gucagcucgu gccgugaggu 1080
   gucggcuuaa gugccauaac gagcgcaacc cuuaucuuua guuacuaaca gguuaugcug 1140
   aggacucuag agagacugcc gucguaagau gugaggaagg uggggaugac gucaaaucag   1200
   cacggcccuu acguccgggg cuacacacgu guuacaaugg gggguacaga aggcagcuag   1260
   cgggugaccg uaugcuaauc ccaaaauccu cucucaguuc ggaucgaagu cugcaacccg   1320
   acuucgugaa gcuggauucg cuaguaaucg cgcaucagcc acggcgcggu gaauacguuc   1380
   ccgggccuug uacacaccgc ccgucaagcc augggagccg gggguaccug aaguacguaa   1440
   ccgcaaggau cguccuaggg uaaaacuggu gacuggggcu aagucguaac aagguagccg   1500
   uaccggaagg ugcggcugga acaccuccuu ucu                                1533
<210> 3
   <211> 1468
   <212> RNA
   <213> Bacteroides ovatus
<220>
   <221> modified_base
   <222> (204)..(207)
   <223> n is a, c, g, or u
<220>
   <221> modified_base
   <222> (339)..(339)
   <223> n is a, c, g, or u
<220>
   <221> modified_base
   <222> (439) .. (439)
   <223> n is a, c, g, or u
<220>
   <221> modified_base
   <222> (530) .. (530)
   <223> n is a, c, g, or u
<220>
   <221> modified_base
   <222> (544) .. (544)
   <223> n is a, c, g, or u
<220>
   <221> modified_base
   <222> (700) .. (700)
   <223> n is a, c, g, or u
<220>
   <221> modified_base
   <222> (739)..(739)
   <223> n is a, c, g, or u
<220>
   <221> modified_base
   <222> (788)..(788)
   <223> n is a, c, g, or u
<220>
   <221> modified_base
   <222> (953)..(953)
   <223> n is a, c, g, or u
<220>
   <221> modified_base
   <222> (962)..(962)
   <223> n is a, c, g, or u
<220>
   <221> modified_base
   <222> (988)..(988)
   <223> n is a, c, g, or u
<220>
   <221> modified_base
   <222> (1031)..(1031)
   <223> n is a, c, g, or u
<220>
   <221> modified_base
   <222> (1084)..(1084)
   <223> n is a, c, g, or u
<400> 3
   augaagaguu ugauccuggc ucaggaugaa cgcuagcuac aggcuuaaca caugcaaguc      60
   gaggggcagc auuuuaguuu gcuugcaaac ugaagauggc gaccggcgca cgggugagua     120
   acacguaucc aaccugccga uaacuccggg auagccuuuc gaaagaaaga uuaauaccgg     180
   aurgyauayg aacaucgcau gaunnnnuua uuaaagaauu ucgguuaucg auggggaugc      240
   guuccauuag uuuguuggcg ggguaacggc ccaccaagac uacgauggau agggguucug     300
   agaggaaggu cccccacauu ggaacugaga cacgguccna acuccuacgg gaggcagcag     360
   ugaggaauau uggucaaugg gcgagagccu gaaccagcca aguagcguga aggaugaagg     420
   cucuaugggu cguaaacunc uuuuauaugg gaauaaagug uuccacgugu ggaauuuugu     480
   auguaccaua ugaauaagga ucggcuaacu ccgugccagc agccgcggun auacggagga     540
   uccnagcguu auccggauuu auuggguuua aagggagcgu agguggauug uuaagucagu     600
   ugugaaaguu ugcggcucaa ccguaaaauu gcaguugaaa cuggcagucu ugaguacagu     660
   agaggugggc ggaauucgug guguagcggu gaaaugcuun gauaucacga agaacuccga     720
   uugcgaaggc agcucacung acuguuacug acacugaugc ucgaaagugu ggguaucaaa     780
   caggauunga uacccuggua guccacacag uaaacgauga auacucgcug uuugcgauau     840
   acaguaagcg gccaagcgaa agcauuaagu auuccaccug gggaguacgc cggcaacggu     900
   gaaacucaaa ggaauugacg ggggcccgca caagcggagg aacauguggu uunauucgau     960
   gnuacgcgag gaaccuuacc cgggcuunaa uugcawcwga auauauwgga aacwruauag    1020
   ccgyaaggca nuugugaagg ugcugcaugg uugucgucag cucgugccgu gaggugucgg    1080
   cuunagugcc auaacgagcg caacccuuau cuuuaguuac uaacagguua ugcugaggac    1140
   ucuagagaga cugccgucgu aagaugugag gaaggugggg augacgucaa aucagcacgg   1200
   cccuuacguc cggggcuaca cacguguuac aauggggggu acagaaggcr gcuaccuggy     1260
   gacaggaugc uaaucccaaa aaccucucuc aguucggauc gaagucugca acccgacuuc    1320
   gugaagcugg auucgcuagu aaucgcgcau cagccauggc gcggugaaua cguucccggg    1380
   ccuuguacac accgcccguc aagccaugaa agccgggggu accugaagua cguaaccgca    1440
   aggagcgucc uaggguaaaa cugguaau                                       1468
<210> 4
   <211> 1524
   <212> RNA
   <213> Bacteroides vulgatus
<220>
   <221> modified_base
   <222> (35) .. (35)
   <223> n is a, c, g, or u
<220>
   <221> modified_base
   <222> (97) .. (97)
   <223> n is a, c, g, or u
<220>
   <221> modified_base
   <222> (99)..(99)
   <223> n is a, c, g, or u
<220>
   <221> modified_base
   <222> (395)..(395)
   <223> n is a, c, g, or u
<220>
   <221> modified_base
   <222> (481) .. (481)
   <223> n is a, c, g, or u
<220>
   <221> modified_base
   <222> (544)..(544)
   <223> n is a, c, g, or u
<220>
   <221> modified_base
   <222> (744)..(744)
   <223> n is a, c, g, or u
<220>
   <221> modified_base
   <222> (932)..(932)
   <223> n is a, c, g, or u
<220>
   <221> modified_base
   <222> (1204)..(1204)
   <223> n is a, c, g, or u
<220>
   <221> modified_base
   <222> (1255)..(1255)
   <223> n is a, c, g, or u
<220>
   <221> modified_base
   <222> (1499)..(1499)
   <223> n is a, c, g, or u
<400> 4
   uauuacaaug aagaguuuga uccuggcuca ggaunaacgc uagcuacagg cuuaacacau      60
   gcaagucgag gggcagcaug gucuuagcuu gcuaagncna uggcgaccgg cgcacgggug     120
   aguaacacgu auccaaccug ccgucuacuc uuggacagcc uucugaaagg aagauuaaua     180
   caagauggca ucaugagucc gcauguucac augauuaaag guauuccggu agacgauggg     240
   gaugcguucc auuagauagu aggcggggua acggcccacc uagucuucga uggauagggg     300
   uucugagagg aagguccccc acauuggaac ugagacacgg uccaaacucc uacgggaggc     360
   agcagugagg aauauugguc aaugggcgag agccngaacc agccaaguag cgugaaggau     420
   gacugcccua uggguuguaa acuucuuuua uaaaggaaua aagucgggua uggauacccg     480
   nuugcaugua cuuuaugaau aaggaucggc uaacuccgug ccagcagccg cgguaauacg     540
   gagnauccga gcguuauccg gauuuauugg guuuaaaggg agcguagaug gauguuuaag     600
   ucaguuguga aaguuugcgg cucaaccgua aaauugcagu ugauacugga uaucuugagu     660
   gcaguugagg caggcggaau ucguggugua gcggugaaau gcuuagauau cacgaagaac     720
   uccgauugcg aaggcagccu gcunagcugc aacugacauu gaggcucgaa agugugggua     780
   ucaaacagga uuagauaccc ugguagucca cacgguaaac gaugaauacu cgcuguuugc     840
   gauauacugc aagcggccaa gcgaaagcgu uaaguauucc accuggggag uacgccggca 900
   acggugaaac ucaaaggaau ugacgggggc cngcacaagc ggaggaacau gugguuuaau 960
   ucgaugauac gcgaggaacc uuacccgggc uuaaauugca gaugaauuac ggugaaagcc 1020
   guaagccgca aggcaucugu gaaggugcug caugguuguc gucagcucgu gccgugaggu 1080
   gucggcuuaa gugccauaac gagcgcaacc cuuguuguca guuacuaaca gguuaugcug 1140
   aggacucuga caagacugcc aucguaagau gugaggaagg uggggaugac gucaaaucag 1200
   cacngcccuu acguccgggg cuacacacgu guuacaaugg gggguacaga gggcngcuac 1260
   cacgcgagug gaugccaauc cccaaaaccu cucucaguuc ggacuggagu cugcaacccg 1320
   acuccacgaa gcuggauucg cuaguaaucg cgcaucagcc acggcgcggu gaauacguuc 1380
   ccgggccuug uacacaccgc ccgucaaguc augggagccg gggguaccug aagugcguaa 1440
   ccgcgaggag cgcccuaggg uaaaacuggu gacuggggcu aagucguaac aagguagcng 1500
   uaccggaagg aacaccuccu uucu 1524
<210> 5
   <211> 1173
   <212> RNA
   <213> Propionibacterium acnes
<400> 5
   uuggagaguu ugauccuggc ucaggacgaa cgcuggcggc gugcuuaaca caugcaaguc 60
   gaacggaaag gcccugcuuu uguggggugc ucgaguggcg aacgggugag uaacacguga 120
   guaaccugcc cuugacuuug ggauaacuuc aggaaacugg ggcuaauacc ggauaggagc 180
   uccugcugca uggugggggu uggaaaguuu cggcgguugg ggauggacuc gcggcuuauc 240
   agcuuguugg ugggguagug gcuuaccaag gcuuugacgg guagccggcc ugagagggug 300
   accggccaca uugggacuga gauacggccc agacuccuac gggaggcagc aguggggaau 360
   auugcacaau gggcggaagc cugaugcagc aacgccgcgu gcgggaugac ggccuucggg 420
   uuguaaaccg cuuucgccug ugacgaagcg ugagugacgg uaauggguaa agaagcaccc 480
   gcuaacuacg ugccagcagc cgcggugaua cguagggugc caacguuguc cggauuuauu 540
   gggcguaaag ggcucguagg ugguugaucg cgucggaagu guaaucuugg ggcuuaaccc 600
   ugagcgugcu uucgauacgg guugacuuga ggaagguagg ggagaaugga auuccuggug 660
   gagcggugga augcgcagau aucaggagga acaccagugg cgaaggcggu ucucugggcc 720
   uuuccugacg cugaggagcg aaagcguggg gagcgaacag gcuuagauac ccugguaguc 780
   cacgcuguaa acggugggua cuaggugugg gguccauucc acggguuccg ugccguagcu 840
   aacgcuuuaa guaccccgcc uggggaguac ggccgcaagg cuaaaacuca aaggaauuga 900
   cggggccccg cacaagcggc ggagcaugcg gauuaauucg augcaacgcg uagaaccuua 960
   ccuggguuug acauggaucg ggagugcuca gagaugggug ugccucuuuu ggggucgguu 1020
   cacagguggu gcauggcugu cgucagcucg ugucgugaga uguuggguua agucccgcaa 1080
   cgagcgcaac ccuuguucuc uguugccagc acguuauggu ggggacucag uggagaccgc 1140
   cggggucaac ucggaggaag guggggauga cgu 1173
<210> 6
   <211> 1517
   <212> RNA
   <213> Clostridium nexile
<220>
   <221> modified_base
   <222> (184)..(185)
   <223> n is a, c, g, or u
<220>
   <221> modified_base
   <222> (341)..(341)
   <223> n is a, c, g, or u
<220>
   <221> modified_base
   <222> (430)..(430)
   <223> n is a, c, g, or u
<400> 6
   gagauuugau ccuggcucag gaugaacgcu ggccggccgu gcuuacacau gcagucgaac 60
   gaagcgcuua aacuggauuu cuucggauug aaguuuuugc ugacugagug gcggacgggu 120
   gaguaacgcg uggguaaccu gccucauaca gggggauaac aguuagaaau gacugcuaau 180
   accnnauaag cgcacagugc ugcauggcac aguguaaaaa cuccggugguaugagaugga 240
   cccgcgucug auuagcuagu ugguggggua acggccuacc aaggcgacga ucaguagccg 300
   gccugagagg gugaacggcc acauugggac ugagacacgg nccaaacucc uacgggaggc 360
   agcagugggg aauauugcac aaugggggaa acccugaugc agcgacgccg cgugagcgaa 420
   gaaguauuun gguauguaaa gcucuaucag cagggaagaa aaugacggua ccugacuaag 480
   aagcaccggc uaaauacgug ccagcagccg cgguaauacg uaggugcaag cguuauccgg 540
   auuuacuggg uguaaaggga gcguagacgg uuguguaagu cugaugugaa agcccggggc 600
   ucaaccccgg acugcauugg aaacuaugua acuagagugu cggagaggua agcggaauuc 660
   cuaguguagc ggugaaaugc guagauauua ggaggaacac caguggcgaa ggcggcuuac 720
   uggacgauca cugacguuga ggcucgaaag cguggggagc aaacaggauu agauacccug 780
   guaguccacg ccguaaacga ugacuacuag gugucgggga gcaaagcucu ucggugccgc 840
   agcaaacgca auaaguaguc caccugggga guacguucgc aagaaugaaa cucaaaggaa 900
   uugacgggga cccgcacaag cguggagcau gugguuuaau ucgagcaacg cgaagaccuu 960
   accuggucuu gacaucccgg ugaccggucc aguaauggga ccuuuccuuc gggacacggu 1020
   gacagguggu gcaugguugu cgucagcucg ugucgugaga uguuggguua agucccgcaa 1080
   cgagcgcaac cccuaucuuc aguagccagc auuuaaggug ggcacucugg agagacugcc 1140
   agggauaacc uggaggaagg uggggaugac gucaaaucau caugccccuu augaccaggg 1200
   cuacacacgu gcuacaaugg cguaaacaaa gggaagcgaa ccugugaggg gaagcaaauc 1260
   ucaaaaauaa cgucucaguu cggauuguag ucugcaacuc gacuacauga agcuggaauc 1320
   gcuaguaauc gcgaaucagc augucgcggu gaauacguuc ccgggucuug uacacaccgc 1380
   ccgucacacc augggaguca guaacgcccg aagucaguga cccaaccgua aggagggagc 1440
   ugccgaaggu gggaccgaua acugggguga agucguaaca agguagccgu aucggaaggu 1500
   gcggcuggau caccucc 1517
<210> 7
   <211> 1480
   <212> RNA
   <213> Eubacterium formicigenerans
<220>
   <221> modified_base
   <222> (1) .. (1)
   <223> n is a, c, g, or u
<220>
   <221> modified_base
   <222> (124) .. (126)
   <223> n is a, c, g, or u
<220>
   <221> modified_base
   <222> (221)..(223)
   <223> n is a, c, g, or u
<220>
   <221> modified_base
   <222> (288)..(288)
   <223> n is a, c, g, or u
<220>
   <221> modified_base
   <222> (350)..(351)
   <223> n is a, c, g, or u
<220>
   <221> modified_base
   <222> (518)..(518)
   <223> n is a, c, g, or u
<220>
   <221> modified_base
   <222> (535) .. (536)
   <223> n is a, c, g, or u
<220>
   <221> modified_base
   <222> (726)..(726)
   <223> n is a, c, g, or u
<220>
   <221> modified_base
   <222> (919)..(919)
   <223> n is a, c, g, or u
<220>
   <221> modified_base
   <222> (922)..(922)
   <223> n is a, c, g, or u
<220>
   <221> modified_base
   <222> (956)..(957)
   <223> n is a, c, g, or u
<220>
   <221> modified_base
   <222> (1185)..(1186)
   <223> n is a, c, g, or u
<400> 7
   nuuaaacgag aguuugaucc uggcucagga ugaacgcugg cggcgugcuu aacacaugca     60
   agucgagcga agcacauaag uuugauucuu cggaugaaga cuuuugugac ugagcggcgg    120
   acgnnngagu aacgcguggg uaaccugccu cauacagggg gauaacagyu agaaauggcu    180
   gcuaauaccg cauaagacca caguacugca ugguacagug nnnaaaacuc cggugguaug    240
   agauggaccc gcgucugauu agguaguugg ugagguaacg gcccaccnag ccgacgauca    300
   guagccgacc ugagagggug accggccaca uugggacuga gacacggccn ngacuccuac    360
   gggaggcagc aguggggaau auugcacaau gggcgaaagc cugaugcagc gacgccgcgu    420
   gaaggaugaa guauuucggu auguaaacuu cuaucagcag ggaagaaaau gacgguaccu    480
   gacuaagaag ccccggcuaa cuacgugcca gcagccgngg uaauacguag ggggnnagcg    540
   uuauccggau uuacugggug uaaagggagc guagacggcu gugcaagucu gaagugaaag 600
   gcaugggcuc aaccugugga cugcuuugga aacugugcag cuagaguguc ggagagguaa 660
   guggaauucc uaguguagcg gugaaaugcg uagauauuag gaggaacacc aguggcgaag 720
   gcggcnuacu ggacgaugac ugacguugag gcucgaaagc guggggagca aacaggauua 780
   gauacccugg uaguccacgc cguaaacgau gacugcuagg ugucggguag caaagcuauu 840
   cggugccgca gcuaacgcaa uaagcagucc accuggggag uacguucgca agaaugaaac 900
   ucaaaggaau ugacggggnc cngcacaagc gguggagcau gugguuuaau ucgaannaac 960
   gcgaagaacc uuaccugauc uugacauccc gaugaccgcu ucguaaugga agyuuuucuu 1020
   cggaacaucg gugacaggug gugcaugguu gucgucagcu cgugucguga gauguugggu 1080
   uaagucccgc aacgagcgca acccuuaucu ucaguagcca gcauuuagga ugggcacucu 1140
   ggagagacug ccagggauaa ccuggaggaa gguggggaug acgunnaauc aucaugcccc 1200
   uuaugaccag ggcuacacac gugcuacaau ggcguaaaca gagggaggca gagccgcgag 1260
   gccgagcaaa ucucaaaaau aacgucucag uucggauugu agucugcaac ucgacuacau 1320
   gaagcuggaa ucgcuaguaa ucgcagauca gaaugcugcg gugaauacgu ucccgggucu 1380
   uguacacacc gcccgucaca ccaugggagu caguaacgcc cgaagucagu gacccaaccg 1440
   aaaggaggga gcugccgaag gugggaccga uaacuggggu 1480
<210> 8
   <211> 1423
   <212> DNA
   <213> Ruminococcus gnavus
<220>
   <221> modified_base
   <222> (1148)..(1148)
   <223> n is a, c, g, or t
<220>
   <221> modified_base
   <222> (1188)..(1188)
   <223> n is a, c, g, or t
<220>
   <221> modified_base
   <222> (1257)..(1257)
   <223> n is a, c, g, or t
<400> 8
   cctggctcag gatgaacgct ggcggcgtgc ttaacacatg caagtcgagc gaagcacctt 60
   gacggatttc ttcggattga agccttggtg actgagcggc ggacgggtga gtaacgcgtg 120
   ggtaacctgc ctcatacagg gggataacag ttggaaacgg ctgctaatac cgcataagcg 180
   cacagtaccg catggtacgg tgtgaaaaac tccggtggta tgagatggac ccgcgtctga 240
   ttaggtagtt ggtggggtaa cggcctacca agccgacgat cagtagccga cctgagaggg 300
   tgaccggcca cattgggact gagacacggc ccaaactcct acgggaggca gcagtgggga 360
   atattgcaca atgggggaaa ccctgatgca gcgacgccgc gtgagcgatg aagtatttcg 420
   gtatgtaaag ctctatcagc agggaagaaa atgacggtac ctgactaaga agccccggct 480
   aactacgtgc cagcagccgc ggtaatacgt agggggcaag cgttatccgg atttactggg 540
   tgtaaaggga gcgtagacgg catggcaagc cagatgtgaa agcccggggc tcaaccccgg 600
   gactgcattt ggaactgtca ggctagagtg tcggagagga aagcggaatt cctagtgtag 660
   cggtgaaatg cgtagatatt aggaggaaca ccagtggcga aggcggcttt ctggacgatg 720
   actgacgttg aggctcgaaa gcgtggggag caaacaggat tagataccct ggtagtccac 780
   gccgtaaacg atgaatacta ggtgtcgggt ggaaaagcca ttcggtgccg cagcaaacgc 840
   aataagtatt ccacctgggg agtacgttcg caagaatgaa actcaaagga attgacgggg 900
   acccgcacaa gcggtggagc atgtggttta attcgaagca acgcgaagaa ccttacctgg 960
   tcttgacatc cctctgaccg ctctttaatc ggagctttcc ttcgggacag aggagacagg 1020
   tggtgcatgg ttgtcgtcag ctcgtgtcgt gagatgttgg gttaagtccc gcaacgagcg 1080
   caacccctat ctttagtagc cagcattttg gatgggcact ctagagagac tvccagggat 1140
   aacctggngg aaggtgggga tgacgtcaaa tcatcatgcc ccttatgncc agggctacac 1200
   acgtgctaca atggcgtaaa caaagggaag cgagcccgcg agggggagca aatcccnaaa    1260
   ataacgtctc agttcggatt gtagtctgca actcgactac atgaagctgg aatcgctagt 1320
   aatcgcgaat cagaatgtcg cggtgaatac gttcccgggt cttgtacaca ccscccgtca 1380
   caccatggga gtmagtaacg cccgaagtca gtgacccaac cgc 1423
<210> 9
   <211> 1418
   <212> DNA
   <213> Ruminococcus torques
<220>
   <221> modified_base
   <222> (724) .. (724)
   <223> n is a, c, g, or t
<220>
   <221> modified_base
   <222> (771)..(771)
   <223> n is a, c, g, or t
<220>
   <221> modified_base
   <222> (776)..(776)
   <223> n is a, c, g, or t
<220>
   <221> modified_base
   <222> (807)..(807)
   <223> n is a, c, g, or t
<400> 9
   ctcaggatga acgctggcgg cgtgcctaac acatgcaagt cgagcgaagc actttgctta     60
   gattcttcgg atgaagagga ttgtgactga gcggcggacg ggtgagtaac gcgtgggtaa    120
   cctgcctcat acagggggat aacagttaga aatgactgct aataccgcat aagaccacag    180
   caccgcatgg tgcgggggta aaaactccgg tggtatgaga tggacccgcg tctgattagc    240
   tagttggtaa ggtaacggct taccaaggcg acgatcagta gccgacctga gagggtgacc    300
   ggccacattg ggactgagac acggcccaaa ctcctacggg aggcagcagt ggggaatatt    360
   gcacaatggg ggaaaccctg atgcagcgac gccgcgtgag cgaagaagta tttcggtatg    420
   taaagctcta tcagcaggga agaaaatgac ggtacctgac taagaagcac cggctaaata    480
   cgtgccagca gccgcggtaa tacgtatggt gcaagcgtta tccggattta ctgggtgtaa    540
   agggagcgta gacggatggg caagtctgat gtgaaaaccc ggggctcaac cccgggactg    600
   cattggaaac tgttcatcta gagtgctgga gaggtaagtg gaattcctag tgtagcggtg    660
   aaatgcgtag atattaggag gaacaccagt ggcgaaggcg gcttactgga cagtaactga    720
   cgtngaggct cgaaagcgtg gggagcacac aggattagat accctggtag nccacnccgt    780
   aaacgatgac tactaggtgt cgggtgncaa agccattcgg tgccgcagca aacgcaataa    840
   gtagtccacc tggggagtac gttcgcaaga atgaaactca aaggaattga cggggacccg    900
   cacaagcggt ggagcatgtg gtttaattcg aagcaacgcg aagaacctta cctgctcttg    960
   acatcccgct gaccggacgg taatgcgtcc ttcccttcgg ggcagcggag acaggtggtg   1020
   catggttgtc gtcagctcgt gtcgtgagat gttgggttaa gtcccgcaac gagcgcaacc   1080
   cctatcttta gtagccagcg gccaggccgg gcactctaga gagactgccg gggataaccc   1140
   ggaggaaggt ggggatgacg tcaaatcatc atgcccctta tgagcagggc tacacacgtg   1200
   ctacaatggc gtaaacaaag ggaagcgaga ccgcgaggtg gagcaaatcc caaaaataac   1260
   gtctcagttc ggattgtagt ctgcaactcg actacatgaa gctggaatcg ctagtaatcg   1320
   cgaatcagaa tgtcgcggtg aatacgttcc cgggtcttgt acacaccgcc cgtcacacca   1380
   tgggagtcag taacgcccga agtcagtgac ccaaccgt                            1418
<210> 10
   <211> 1458
   <212> RNA
   <213> Clostridium sp.
<400> 10
   gaugaacgcu ggcggcgugc uuaacacaug caagucgagc gaagcgauuc uaawgaaguu     60
   uucggaygga auuuraauug acugagcggc ggacggguga guaacgcgug gguaaccugc    120
   cucauacagg gggauaacag uuggaaacgg cugcuaauac cgcauaagca cacagugccg    180
   caugguacgg ugugaaaaac uccgguggua ugagauggac ccgcgucuga uuagguaguu    240
   ggugagguaa cggcccacca agccgacgau caguagccga ccugagaggg ugaccggcca    300
   cauugggacu gagacacggc ccaaacuccu acgggaggca gcagugggga auauuggaca    360
   augggggaaa cccugaucca gcgacgccgc gugagugaag aaguauuucg guauguaaag    420
   cucuaucagc agggaagaaa augacgguac cugacuaaga agccccggcu aacuacgugc    480
   cagcagccgc gguaauacgu agggggcaag cguuauccgg auuuacuggg uguaaaggga    540
   gcguagacgg caaugcaagu cuggagugaa agcccggggc ucaaccccgg gacugcuuug    600
   gaaacugugu ugcuagagug caggagaggu aaguggaauu ccuaguguag cggugaaaug    660
   cguagauauu aggaggaaca ccaguggcga aggcggcuua cuggacugua acugacguug    720
   aggcucgaaa gcguggggag caaacaggau uagauacccu gguaguccac gccguaaacg    780
   augaauacua gguguugggg agcaaagcuc uucggugccg ccgcuaacgc aauaaguauu    840
   ccaccugggg aguacguucg caagaaugaa acucaaagga auugacgggg acccgcacaa    900
   gcgguggagc augugguuua auucgaagca acgcgaagaa ccuuaccaag ucuugacauc    960
   ggaaugaccg guccguaacg gggccuuccc uacggggcau uccagacagg uggugcaugg   1020
   uugucgucag cucgugucgu gagauguugg guuaaguccc gcaacgagcg caacccuuau   1080
   ccuuaguagc cagcauguag uggugggcac ucuggggaga cugccaggga uaaccuggag   1140
   gaaggugggg augacgucaa aucaucaugc cccuuaugau uugggcuaca cacgugcuac   1200
   aauggcguaa acaaagggaa gcaaaggagc gaucuuaagc aaaccccaaa aauaacgucu   1260
   caguucggau uguagucugc aacucgacua caugaagcug gaaucgcuag uaaucgcgga   1320
   ucagaaugcc gcggugaaua cguucccggg ucuuguacac accgcccguc acaccauggg   1380
   aguugguaac gcccgaaguc agugacccam ccsymaggag ggagcugccg aaggcgggac   1440
   urauaacugg ggugaaug                                                 1458
<210> 11
   <211> 728
   <212> RNA
   <213> Moraxella cuniculi
<400> 11
   aggcuuaaca caugcaaguc gaacgaaguu agggagcuug cuccugauac uuaguggcgg 60
   acgggugagu aaugcuuagg aaucugccua guaguggggg auaacuaucc gaaaggauag 120
   cuaauaccgc auacgaccua cgggugaaag ggggcguaag cucucgcuau uagaugagcc 180
   uaagucggau uagcuaguug gugggguaaa ggccuaccaa ggcgacgauc uguagcuggu 240
   cugagaggau gaucagccac acugggacug agacacggcc cagacuccua cgggaggcag 300
   caguggggaa uauuggacaa ugggcgaaag ccugauccag ccaugcccgc gugugugaag 360
   aaggccuuuu gguuguaaag cacuuuaagu ggggaggaaa agcuaauagc uaauaccuau 420
   uagcccugac guuacccaca gaauaagcac cggcuaacuc ugugccagca gccgcgguaa 480
   uacagagggu gcaagcguua aucggaauua cugggcguaa agcgcgcgua ggugguuacu 540
   uaagucagau gugaaagccc cgggcuuaac cugggaacug caucugauac uggguaacua 600
   gaguagguga gagggaagua gaauuccagg uguagcggug aaaugcguag agaucuggag 660
   gaauaccgau ggcgaaggca gcuuccuggc aucauacuga cacugaggug cgaaagcgug 720
   gguagcaa 728
<210> 12
   <211> 1519
   <212> RNA
   <213> Moraxella lacunata
<400> 12
   agaguuugau cauggcucag auugaacgcu ggcggcaggc uuaacacaug caagucgaac 60
   gaugaagucu agcuugcuag acggauuagu ggcgaacggg ugaguaaugc uuaggaaucu 120
   gccuauuagu gggggauaac guagggaaac uuacgcuaau accgcauacg ucuuacgaga 180
   gaaagggggc uuuuagcucu cgcuaauaga ugagccuaag ucggauuagc uaguuggugg 240
   gguaaaggcc uaccaaggcg acgaucugua gcuggucuga gaggaugauc agccacacug 300
   ggacugagac acggcccaga cuccuacggg aggcagcagu ggggaauauu ggacaauggg 360
   cgaaagccug auccagccau gccgcgugug ugaagaaggc cuuuugguug uaaagcacuu 420
   uaagugggga ggaaaagcuu gugguuaaua ccuacaagcc cugacguuac ccacagaaua 480
   agcaccggcu aacucugugc cagcagccgc gguaauacag agggugcaag cguuaaucgg    540
   aauuacuggg cguaaagcga gcguaggugg ucauuuaagu cagaugugaa agccccgggc    600
   uuaaccuggg aacugcaucu gauacugggu gacuagagua ggugagaggg aaguagaauu    660
   ccagguguag cggugaaaug cguagagauc uggaggaaua ccgauggcga aggcagcuuc    720
   cuggcaucau acugacacug agguucgaaa gcguggguag caaacaggau uagauacccu    780
   gguaguccac gccguaaacg augucuacca gucguugggu cucuugaaga cuuagugacg    840
   caguuaacgc aauaaguaga ccgccugggg aguacggccg caagguuaaa acucaaauga    900
   auugacgggg cccgcacaag cgguggagca ugugguuuaa uucgaugcaa cgcgaagaac    960
   cuuaccuggu cuugacauag ugagaauccu gcagagaugc gggagugcuu cgggaauuca   1020
   cauacaggug cugcauggcu gucgucagcc cgugucguga gauguugggu uaagucccgc   1080
   aacgagcgca acccuuuucc uuaguuacca gcgauuuaag ucgggaacuc uaaggauacu   1140
   gccagugaca aacuggagga aggcgggacg acgucaaguc aucauggccc uuacgaccag   1200
   ggcuacacac gugcuacaau gguugguaca aaggguugcu acacagcgau gugaugcuaa   1260
   ucucaaaaag ccaaucguag uccggauugg agucugcaac ucgacuccau gaagucggaa   1320
   ucgcuaguaa ucgcagauca gaaugcugcg gugaauacgu ucccgggccu uguacacacc   1380
   gcccgucaca ccaugggagu ugaucucacc agaagugguu agccuaacgc aagagggcga   1440
   ucaccacggu ggggucgaug acugggguga agucguaaca agguagccgu aggggaacug   1500
   cgguuggauc accuccuua                                                1519
<210> 13
   <211> 1448
   <212> RNA
   <213> Moraxella osloensis
<400> 13
   cuggcggcag gcuuaacaca ugcaagucga acgaugacuc ucuagcuugc uagagaugau     60
   uaguggcgga cgggugagua acauuuagga aucugccuag uaguggggga uagcucgggg    120
   aaacucgaau uaauaccgca uacgaccuac gggugaaagg gggcgcaagc ucuugcuauu    180
   agaugagccu aaaucagauu agcuaguugg ugggguaaag gcccaccaag gcgacgaucu   240
   guaacugguc ugagaggaug aucagucaca ccggaacuga gacacggucc ggacuccuac    300
   gggaggcagc aguggggaau auuggacaau gggggcaacc cugauccagc caugccgcgu    360
   gugugaagaa ggccuuuugg uuguaaagca cuuuaagcag ggaggagagg cuaaugguua 420
   auacccauua gauuagacgu uaccugcaga auaagcaccg gcuaacucug ugccagcagc 480
   cgcgguaaua cagagggugc gagcguuaau cggaauuacu gggcguaaag cgaguguagg 540
   uggcucauua agucacaugu gaaauccccg ggcuuaaccu gggaacugca ugugauacug 600
   guggugcuag aauaugugag agggaaguag aauuccaggu guagcgguga aaugcguaga 660
   gaucuggagg aauaccgaug gcgaaggcag cuuccuggca uaauauugac acugagauuc 720
   gaaagcgugg guagcaaaca ggauuagaua cccugguagu ccacgccgua aacgaugucu 780
   acuagccguu gggguccuug agacuuuagu ggcgcaguua acgcgauaag uagaccgccu 840
   ggggaguacg gccgcaaggu uaaaacucaa augaauugac gggggcccgc acaagcggug 900
   gagcaugugg uuuaauucga ugcaacgcga agaaccuuac cuggucuuga cauagugaga 960
   aucucucaga gaugagagag ugccuucggg aacucacaua caggugcugc auggcugucg 1020
   ucagcucgug ucgugagaug uuggguuaag ucccgcaacg agcgcaaccc uuuuccuuau 1080
   uugccagcgg guuaagccgg gaacuuuaag gauacugcca gugacaaacu ggaggaaggc 1140
   ggggacgacg ucaagucauc auggcccuua cgaccagggc uacacacgug cuacaauggu 1200
   agguacagag gguugcuaca cagcgaugug augcuaaucu caaaaagccu aucguagucc 1260
   ggauuggagu cugcaacucg acuccaugaa gucggaaucg cuaguaaucg cggaucagaa 1320
   ugccgcggug aauacguucc cgggccuugu acacaccgcc cgucacacca ugggagucua 1380
   uugcaccaga aguagguagc cuaacgaaag agggcgcuua ccacggugug gucgaugacu 1440
   ggggugaa 1448
<210> 14
   <211> 452
   <212> RNA
   <213> Acinetobacter junii
<400> 14
   caugcaaguc gagcggagau gaggugcuug caccuuaucu uagcggcgga cgggugagua 60
   augcuuagga aucugccuau uaguggggga caacauuccg aaaggaaugc uaauaccgca 120
   uacguccuac gggagaaagc aggggaucuu cggaccuugc gcuaauagau gagccuaagu 180
   cggauuagcu aguugguggg guaaaggccu accaaggcga cgaucuguag cgggucugag 240
   aggaugaucc gccacacugg gacugagaca cggcccagac uccuacggga ggcagcagug 300
   gggaauauug gacaaugggg ggaacccuga uccagccaug ccgcgugugu gaagaaggcc 360
   uuaugguugu aaagcacuuu aagcgaggag gaggcuacug agacuaauac ucuuggauag 420
   uggacguuac ucgcagaaua agcaccggcu aa 452
<210> 15
   <211> 1488
   <212> RNA
   <213> Comamonas sp.
<400> 15
   auugaacgcu ggcggcaugc cuuacacaug caagucgaac gguaacaggu cuucggaugc      60
   ugacgagugg cgaacgggug aguaauacau cggaacgugc ccgauygugg gggauaacga     120
   ggcgaaagcu uugcuaauac cgcauacgau cuacggauga aagcggggga ucuucggacc     180
   ucgcgcggac ggagcggccg auggcagauu agguaguugg ugggauaaaa gcuuaccaag     240
   ccgacgaucu guagcugguc ugagaggaug aucagccaca cugggacuga gacacggccc     300
   agacuccuac gggaggcagc aguggggaau uuuggacaau gggggaaacc cugauccagc     360
   caugccgcgu gcaggaugaa ggccuucggg uuguaaacug cuuuuguacg gaacgaaaag     420
   gucucuucua auaaaggggg cccaugacgg uaccguaaga auaagcaccg gcuaacuacg     480
   ugccagcagc cgcgguaaua cguagggugc aagcguuaau cggaauuacu gggcguaaag     540
   cgugcgcagg cgguuaugua agacagaugu gaaauccccg ggcucaaccu gggaacugca     600
   uuugugacug cauggcuuga gugcggcaga gggggaugga auuccgcgug uagcagugaa     660
   augcguagau augcggagga acaccgaugg cgaaggcaau ccccugggcc ugcacugacg     720
   cucaugcacg aaagcguggg gagcaaacag gauuagauac ccugguaguc cacgcccuaa     780
   acgaugucaa cugguuguug ggaauuuguu uucucaguaa cgaagcuaac gcgugaaguu     840
   gaccgccugg ggaguacggc cgcaagguug aaacucaaag gaauugacgg ggacccgcac     900
   aagcggugga ugaugugguu uaauucgaug caacgcgaaa aaccuuaccc accuuugaca     960
   uggcaggaag uccacagaga ugaggaugug cucgaaagag aaccugcaca caggugcugc    1020
   auggcugucg ucagcucgug ucgugagaug uuggguuaag ucccgcaacg agcgcaaccc    1080
   uugucauuag uugcuacauu uaguugggca cucuaaugag acugccggug acaaaccgga    1140
   ggaagguggg gaugacguca aguccucaug gcccuuauag guggggcuac acacgucaua    1200
   caauggcugg uacaaagggu ugccaacccg cgagggggag cuaaucccau aaagccaguc    1260
   guaguccgga ucgcagucug caacucgacu gcgugaaguc ggaaucgcua guaaucgugg    1320
   aucagaaugu cacggugaau acguucccgg gucuuguaca caccgcccgu cacaccaugg    1380
   gagcgggucu cgccagaagu agguagccua accgcaagga gggcgcuuac cacggcgggg    1440
   uucgugacug gggugaaguc guaacaaggu agccguaucg gaaggugc                 1488
<210> 16
   <211> 1520
   <212> RNA
   <213> Comamonas terrigena
<220>
   <221> modified_base
   <222> (623)..(623)
   <223> n is a, c, g, or u
<220>
   <221> modified_base
   <222> (630)..(630)
   <223> n is a, c, g, or u
<400> 16
   aguuugaucc uggcucagau ugaacgcugg cggcaugcuu uacacaugca agucgaacgg     60
   cagcacggac uucggucugg uggcgagugg cgaacgggug aguaauacau cggaacgugc    120
   ccaguugugg gggauaacua cucgaaagag uagcuaauac cgcaugagua cugagguuga    180
   aagcagggga ucgcaagacc uugcgcaacu ggagcggccg auggcagauu agguaguugg    240
   ugggauaaaa gcuuaccaag ccgacgaucu guagcugguc ugagaggacg accagccaca    300
   cugggacuga gacacggccc agacuccuac gggaggcagc aguggggaau uuuggacaau    360
   gggcgaaagc cugauccagc aaugccgcgu gcaggaugaa ggccuucggg uuguaaacug    420
   cuuuuguacg gaacgaaaag cuucggguua auaccuggag ucaugacggu acccuaagaa    480
   uaagcaccgg cuaacuacgu gccagcagcc gcgguaauac guagggugca agcguuaauc    540
   ggaauuacug ggcguaaagc gugcgcaggc ggucuuguaa gacagaggug aaauccccgg    600
   gcucaaccug ggaacugccu uunugacugn aaggcuggag ugcggcagag ggggauggaa    660
   uuccgcgugu agcagugaaa ugcguagaua ugcggaggaa caccgauggc gaaggcaauc    720
   cccugggccu gcacugacgc ucaugcacga aagcgugggg agcaaacagg auuagauacc    780
   cugguagucc acggccuaaa cgaugucaac ugguuguugg gucuuaacug acucaguaac    840
   gaagcuaacg cgugaaguug accgccuggg gaguacggcc gcaagguuga aacucaaagg    900
   aauugacggg gacccgcaca agcgguggau gaugugguuu aauuugaugc aacgcgaaaa    960
   accuuaccca ccuuugacau guacggaauc cuuuagagau agaggagugc ucgaaagaga   1020
   gccguaacac aggugcugca uggcugucgu cagcucgugu cgugagaugu uggguuaagu   1080
   cccgcaacga gcgcaacccu ugccauuagu ugcuacgaaa gggcacucua augggacuuc   1140
   cggugacaaa ccggaggaag guggggauga cgucaagucc ucauggcccu uauagguggg    1200
   gcuacacacg ucauacaaug gcugguacaa aggguugcca acccgcgagg gggagcuaau   1260
   cccauaaagc cagucguagu ccgguucgca gucugcaacu cgacugcgug aagucggaau   1320
   cgcuaguaau cguggaucag aaugucacgg ugaauacguu cccgggucuu guacacaccg   1380
   cccgucacac caugggagcg ggucucgcca gaaguaggua gccuaaccgu aaggagggcg   1440
   cuuaccacgg cgggguucgu gacuggggug aagucguaac aagguagccg uaucggaagg   1500
   ugcggcugga ucaccuccuu                                                  1520
<210> 17
   <211> 1476
   <212> RNA
   <213> Bergeyella zoohelcum
<220>
   <221> modified_base
   <222> (138)..(138)
   <223> n is a, c, g, or u
<220>
   <221> modified_base
   <222> (141) .. (143)
   <223> n is a, c, g, or u
<220>
   <221> modified_base
   <222> (183)..(183)
   <223> n is a, c, g, or u
<220>
   <221> modified_base
   <222> (278) .. (278)
   <223> n is a, c, g, or u
<220>
   <221> modified_base
   <222> (285) .. (285)
   <223> n is a, c, g, or u
<220>
   <221> modified_base
   <222> (317)..(318)
   <223> n is a, c, g, or u
<220>
   <221> modified_base
   <222> (341) .. (342)
   <223> n is a, c, g, or u
<220>
   <221> modified_base
   <222> (344)..(344)
   <223> n is a, c, g, or u
<220>
   <221> modified_base
   <222> (528) .. (528)
   <223> n is a, c, g, or u
<220>
   <221> modified_base
   <222> (532)..(533)
   <223> n is a, c, g, or u
<220>
   <221> modified_base
   <222> (542)..(542)
   <223> n is a, c, g, or u
<220>
   <221> modified_base
   <222> (547)..(547)
   <223> n is a, c, g, or u
<220>
   <221> modified_base
   <222> (737)..(737)
   <223> n is a, c, g, or u
<220>
   <221> modified_base
   <222> (766)..(767)
   <223> n is a, c, g, or u
<220>
   <221> modified_base
   <222> (965)..(966)
   <223> n is a, c, g, or u
<220>
   <221> modified_base
   <222> (1022)..(1022)
   <223> n is a, c, g, or u
<220>
   <221> modified_base
   <222> (1085)..(1086)
   <223> n is a, c, g, or u
<220>
   <221> modified_base
   <222> (1096)..(1096)
   <223> n is a, c, g, or u
<220>
   <221> modified_base
   <222> (1205)..(1205)
   <223> n is a, c, g, or u
<220>
   <221> modified_base
   <222> (1288)..(1289)
   <223> n is a, c, g, or u
<220>
   <221> modified_base
   <222> (1384)..(1384)
   <223> n is a, c, g, or u
<220>
   <221> modified_base
   <222> (1406)..(1406)
   <223> n is a, c, g, or u
<400> 17
   auacaaugga gaguuugauc cuggcucagg augaacgcua gcgggaggcc uaacacaugc 60
   aagccgagcg ggauuuguug guuagcuugc uaacuaacaa ugagagcggc guacgggugc 120
   guaacacgug ugcaaccngc nnnuaucugg gggauagccu uucgaaagga agauuaauac 180
   ccnauaauau auugauuggc aucaguuaau auugaaagcu ccggcggaua gagaugggca 240
   cgcguaagau uagcuaguug gugagguaac ggcucacnaa ggcuncgauc uuuagggggc 300
   cugagagggu gaucccnnac acugguacug agacacggac nngncuccua cgggaggcag 360
   cagugaggaa uauuggacaa ugggugagag ccugauccag ccaucccgcg ugaaggacua 420
   aggaccuaug guuuguaaac uucuuuuaua cagggauaaa ccuacucucg ugaggguagc 480
   ugaagguacu guaugaauaa gcaccggcua acuccgugcc agcagccncg gnnauacgga 540
   gngugcnagc guuauccgga uuuauugggu uuaaaggguc cguaggcggg ucgauaaguc 600
   aguggugaaa gccugcagcu uaacuguaga acugccguug auacugucgg ucuugagugu 660
   auuugaggua gcuggaauga guaguguagc ggugaaaugc auagauauua cucagaacac 720
   caauugcgaa ggcaggnuac caaguuacaa cugacgcuga uggacnnaag cguggggagc 780
   gaacaggauu agauacccug guaguccacg cuguaaacga ugcuaacucg uuuuuggggu 840
   auuauacuuc agagaccaag cgaaagugau aaguuagcca ccuggggagu acgaacgcaa 900
   guuugaaacu caaaggaauu gacgggggcc cgcacaagcg guggauuaug ugguuuaauu 960
   cgaunnuacg cgaggaaccu uaccaagacu uaaaugggaa uugacagcug uagaaauacg 1020
   gnuuucuucg gacaauuuuc aaggugcugc augguugucg ucagcucgug ccgugaggug 1080
   uuagnnuaag uccugnaacg agcgcaaccc cugucacuag uugccaucau uaaguugggg 1140
   acucuaguga gacugccuac gcaaguagag aggaaggugg ggaugacguc aaaucaucac 1200
   ggccnuuacg ucuugggcca cacacguaau acaauggccg guacagaggg cagcuacacu 1260
   gcgaagugau gcgaaucucg aaagccgnnc ucaguucgga uuggagucug caacucgacu 1320
   cuaugaagcu ggaaucgcua guaaucgcgc aucagccaug gcgcggugaa uacguucccg 1380
   ggcnuuguac acaccgcccg ucaagncaug gaagucuggg guaccugaag ucggugaccg 1440
   uuaaaggagc ugccuagggu aaaacaggua acuagg 1476
<210> 18
   <211> 395
   <212> RNA
   <213> Chryseobacterium balustinum str. SBR1044
<220>
   <221> modified_base
   <222> (343) .. (343)
   <223> n is a, c , g, or u
<400> 18
   uagcgggagg gcuaacacau gcaagccgag cgguauuguu ucuucggaaa ugagagagcg 60
   gcguacgggu gcggaacacg ugugcaaccu gccuuuaucu gggggauagc cuuucgaaag 120
   gaagauuaau acuccauaac auauugaacg gcaucguuug auauugaaag cuccggcgga 180
   uagagauggg cacgcgcaag auuagcuagu uggugaggua acggcucacc aaggcgauga 240
   ucuuuagggg ggcugagagg gugauccccc acacugguac ugagacacgg accagacucc 300
   uacgggaggc agcagugagg aauauugguc aaugggugca agncugaacc agccaucccg 360
   cgugaaggac gacugcccua uggguuguaa acuuc 395
<210> 19
   <211> 395
   <212> RNA
   <213> Chryseobacterium balustinum str. SBR2024
<220>
   <221> modified_base
   <222> (11) .. (11)
   <223> n is a, c, g, or u
<220>
   <221> modified_base
   <222> (14) .. (14)
   <223> n is a, c, g, or u
<220>
   <221> modified_base
   <222> (17)..(18)
   <223> n is a, c, g, or u
<220>
   <221> modified_base
   <222> (310)..(310)
   <223> n is a, c, g, or u
<400> 19
   uagcgggagg ncunacnnau gcaagccgag cgguauuguu ucuucggaaa ugagagagcg 60
   gcguacgggu gcggaacacg ugugcaaccu gccuuuaucu gggggauagc cuuucgaaag 120
   gaagauuaau acuccauaac auauugauug gcaucaauua auauugaaag cuccggcgga 180
   uagagauggg cacgcgcaag auuagcuagu uggugaggua acggcucacc aaggcgauga 240
   ucuuuagggg ggcugagagg gugauccccc acacugguac ugagacacgg accagacucc 300
   uacgggaggn agcagugagg aauauugguc aaugggugca agccugaacc agccaucccg 360
   cgugaaggac gacugcccua uggguuguaa acuuc 395
<210> 20
   <211> 1435
   <212> RNA
   <213> Clostridium paraputrificum
<400> 20
   cgaacgcugg cggcgugccu aacacaugca agucgagcga ugaaguuccu ucgggaacgg     60
   auuagcggcg gacgggugag uaacacgugg gcaaccugcc uuauagaggg gaauagccuu    120
   ccgaaaggaa gauuaauacc gcauaagauu guagcuucgc augaaguagc aauuaaagga    180
   gcaauccgcu auaagauggg cccgcggcgc auuagcuagu uggugaggua acggcucacc    240
   aaggcgacga ugcguagccg accugagagg gugaucggcc acauugggac ugagacacgg    300
   cccagacucc uacgggaggc agcagugggg aauauugcac aaugggggaa acccugaugc    360
   agcaacgccg cgugagugau gacggccuuc ggguuguaaa gcucugucuu uggggacgau    420
   aaugacggua cccaaggagg aagccacggc uaacuacgug ccagcagccg cgguaauacg    480
   uagguggcaa gcguuguccg gauuuacugg gcguaaaggg agcguaggcg gauuuuuaag    540
   ugggauguga aauacccggg cucaaccugg gugcugcauu ccaaacugga aaucuagagu    600
   gcaggagggg aaaguggaau uccuagugua gcggugaaau gcguagagau uaggaagaac    660
   accaguggcg aaggcgacuu ucuggacugu aacugacgcu gaggcucgaa agcgugggga    720
   gcaaacagga uuagauaccc ugguagucca cgccguaaac gaugaauacu agguguaggg    780
   guugucauga ccucugugcc gccgcuaacg cauuaaguau uccgccuggg gaguacgguc    840
   gcaagauuaa aacucaaagg aauugacggg ggcccgcaca aguagcggag caugugguuu    900
   aauucgaagc aacgcgaaga accuuaccua gacuugacau cuccugaauu accauguaau    960
   gugggaaguc ccuucgggga caggaagaca gguggugcau gguugucguc agcucguguc   1020
   gugagauguu ggguuaaguc ccgcaacgag cgcaacccuu auuguuaguu gcuaccauuu   1080
   aguugagcac ucuagcgaga cugcccgggu uaaccgggag gaaggugggg augacgucaa   1140
   aucaucaugc cccuuauguc uagggcuaca cacgugcuac aauggccggu acaacgagau   1200
   gcaauaccgu gagguggagc aaaacuauaa aaccggucuc aguucggauu guaggcugaa   1260
   acucgccuac augaagcugg aguuacuagu aaucgcgaau cagaaugucg cggugaauac   1320
   guucccgggc cuuguacaca ccgcccguca caccaugaga guuggcaaua cccaaaguug   1380
   gugaucuaac ccguaaggga ggaagccacc uaagguaggg ucagcgauug gggug         1435
<210> 21
   <211> 1509
   <212> RNA
   <213> Enterococcus cecorum
<400> 21
   gacgaacgcu ggcggcgugc cuaauacaug caagucgaac gcauuuucuu ucaccguagc 60
   uugcuacacc ggaagaaaau gaguggcgaa cgggugagua acacgugggu aaccugccca 120
   ucagcggggg auaacacuug gaaacaggug cuaauaccgc auaauuccau uuaccgcaug 180
   guagauggau gaaaggcgcu uuugcgucac ugauggaugg acccgcggug cauuagcuag 240
   uugguggggu aacggccuac caaggcugcg augcauagcc gaccugagag ggugaucggc 300
   cacacuggga cugagacacg gcccagacuc cuacgggagg cagcaguagg gaaucuucgg 360
   caauggacgc aagucugacc gagcaacgcc gcgugaguga agaagguuuu cggaucguaa 420
   aacucuguug uuagagaaga acaaggauga gaguggaaag uucaucccuu gacgguaucu 480
   aaccagaaag ccacggcuaa cuacgugcca gcagccgcgg uaauacguag guggcaagcg 540
   uuguccggau uuauugggcg uaaagcgagc gcaggcgguc uuuuaagucu gaugugaaag 600
   cccccggcuu aaccggggag ggucauugga aacugggaga cuugagugca gaagaggaaa 660
   gcggaauucc auguguagcg gugaaaugcg uagauauaug gaggaacacc aguggcgaag 720
   gcggcuuucu ggucuguaac ugacgcugag gcucgaaagc guggggagca aacaggauua 780
   gauacccugg uaguccacgc cguaaacgau gagugcuaag uguuggaggg uuuccgcccu 840
   ucagugcugc agcaaacgca uuaagcacuc cgccugggga guacgaccgc aagguugaaa 900
   cucaaaggaa uugacgggga cccgcacaag cgguggagca ugugguuuaa uucgaagcaa 960
   cgcgaagaac cuuaccaggu cuugacaucc uuugaccauc cuagagauag gauuuucccu 1020
   ucggggacaa agugacaggu ggugcauggu ugucgucagc ucgugucgug agauguuggg 1080
   uuaagucccg caacgagcgc aacccuuauu guuaguugcc aucauucagu ugggcacucu 1140
   agcgagacug ccgcagacaa ugcggaggaa gguggggaug acgucaaauc aucaugcccc 1200
   uuaugaccug ggcuacacac gugcuacaau ggagaguaca acgagucgca aagccgcgag 1260
   gcuaagccaa ucucuuaaag cucuucucag uucggauugu aggcugcaac ucgccuacau 1320
   gaagccggaa ucgcuaguaa ucgcggauca gcacgccgcg gugaauacgu ucccgggucu 1380
   uguacacacc gcccgucaca ccacgagagu uuguaacacc caaagccggu gcgguaaccg 1440
   caaggagcca gccgucuaag gugggauaga ugauuggggu gaagucguaa caagguagcc 1500
   guaucggaa 1509
<210> 22
   <211> 1493
   <212> RNA
   <213> Enterococcus columbae
<220>
   <221> modified_base
   <222> (33)..(33)
   <223> n is a, c, g, or u
<220>
   <221> modified_base
   <222> (103)..(103)
   <223> n is a, c, g, or u
<220>
   <221> modified_base
   <222> (158)..(158)
   <223> n is a, c, g, or u
<220>
   <221> modified_base
   <222> (194)..(196)
   <223> n is a, c, g, or u
<220>
   <221> modified_base
   <222> (209) .. (209)
   <223> n is a, c, g, or u
<220>
   <221> modified_base
   <222> (230)..(230)
   <223> n is a, c, g, or u
<220>
   <221> modified_base
   <222> (254)..(254)
   <223> n is a, c, g, or u
<220>
   <221> modified_base
   <222> (289)..(289)
   <223> n is a, c, g, or u
<220>
   <221> modified_base
   <222> (316)..(316)
   <223> n is a, c, g, or u
<220>
   <221> modified_base
   <222> (346)..(347)
   <223> n is a, c, g, or u
<220>
   <221> modified_base
   <222> (367)..(367)
   <223> n is a, c, g, or u
<220>
   <221> modified_base
   <222> (430)..(432)
   <223> n is a, c, g, or u
<220>
   <221> modified_base
   <222> (441) .. (441)
   <223> n is a, c, g, or u
<220>
   <221> modified_base
   <222> (492) .. (492)
   <223> n is a, c, g, or u
<220>
   <221> modified_base
   <222> (555)..(556)
   <223> n is a, c, g, or u
<220>
   <221> modified_base
   <222> (565)..(565)
   <223> n is a, c, g, or u
<220>
   <221> modified_base
   <222> (613)..(613)
   <223> n is a, c, g, or u
<220>
   <221> modified_base
   <222> (639)..(639)
   <223> n is a, c, g, or u
<220>
   <221> modified_base
   <222> (783)..(783)
   <223> n is a, c, g, or u
<220>
   <221> modified_base
   <222> (788)..(788)
   <223> n is a, c, g, or u
<220>
   <221> modified_base
   <222> (792)..(792)
   <223> n is a, c, g, or u
<220>
   <221> modified_base
   <222> (811) .. (811)
   <223> n is a, c, g, or u
<220>
   <221> modified_base
   <222> (871)..(871)
   <223> n is a, c, g, or u
<220>
   <221> modified_base
   <222> (898)..(898)
   <223> n is a, c, g, or u
<220>
   <221> modified_base
   <222> (965)..(965)
   <223> n is a, c, g, or u
<220>
   <221> modified_base
   <222> (979)..(979)
   <223> n is a, c, g, or u
<220>
   <221> modified_base
   <222> (1013)..(1013)
   <223> n is a, c, g, or u
<220>
   <221> modified_base
   <222> (1035)..(1037)
   <223> n is a, c, g, or u
<220>
   <221> modified_base
   <222> (1063)..(1063)
   <223> n is a, c, g, or u
<220>
   <221> modified_base
   <222> (1068)..(1068)
   <223> n is a, c, g, or u
<220>
   <221> modified_base
   <222> (1071)..(1071)
   <223> n is a, c, g, or u
<220>
   <221> modified_base
   <222> (1102)..(1102)
   <223> n is a, c, g, or u
<220>
   <221> modified_base
   <222> (1127) .. (1128)
   <223> n is a, c, g, or u
<220>
   <221> modified_base
   <222> (1228)..(1228)
   <223> n is a, c, g, or u
<220>
   <221> modified_base
   <222> (1337)..(1337)
   <223> n is a, c, g, or u
<220>
   <221> modified_base
   <222> (1399)..(1399)
   <223> n is a, c, g, or u
<220>
   <221> modified_base
   <222> (1401)..(1401)
   <223> n is a, c, g, or u
<220>
   <221> modified_base
   <222> (1424) .. (1424)
   <223> n is a, c, g, or u
<220>
   <221> modified_base
   <222> (1462)..(1462)
   <223> n is a, c, g, or u
<400> 22
   ugagaguuug auccuggcuc aggacgaacg cungcggcgu gccuaauaca ugcaagucga 60
   acgcacuuuc uuucaccgua gcuugcuaca ccgaaaguaa gunaguggcg aacgggugag 120
   uuacacgugg guaaccugcc caucagcggg ggauaacncu uggaaacagg ugcuaauacc 180
   gcauaauauu acunnncgca ugagaaguna uugaaaggcg caacugcgun acugauggau 240
   ggacccgcgg ugcnuuagcu aguuggugag guaacggccu accaaggcna cgaugcauag 300
   ccgaccugag agggunaucg gccacacugg gacugagaca cggccnnaac uccuacggga 360
   ggcagcngua gggaaucuuc ggcaauggac gcaagucuga ccgagcaacg ccgcgugagu 420
   gaagaaggun nncggaucgu naaacucugu uguuagagaa gaacagggau gagaguggaa 480
   aguucauccc ungacgguau cuaaccagaa agccacggcu aacuacgugc cagcagccgc 540
   gguaauacgu aggunncaag cguunuccgg auuuauuggg cguaaagcga gcgcaggcgg 600
   ucuuuuaagu cunaugugaa agcccacggc uuaaccgung agggucauug gaaacuggga 660
   gacuugagug cagaagagga aagcggaauu ccauguguag cggugaaaug cguagauaua 720
   ugcaggaaca ccaguggcga aggcggcuuu cuggucugua acugacgcug aggcucgaaa 780
   gcnugggnag gnaacaggau uagauacccu nguaguccac gccguaaacg augagugcua 840
   aguguuggag gguuuccgcc cuucagugcu ncagcaaacg cauuaagcac uccgccungg 900
   gaguacgacc gcaagguuga aacucaaagg aauugacggg gaccgcacaa gcgguggagc 960
   augunguuua auucgaagna acgcgaagaa ccuuaccagg ucuugacauc cunugaccau 1020
   ccuagagaua ggacnnnccu ucggggacaa agugacaggu ggngcaungu ngucgucagc 1080
   ucgugucgug agauguuggg unaagucccg caacgagcgc aacccunnuu guuaguugcc 1140
   aucauuuagu ugggcacucu agcgagacug ccgcagacaa ugcggaggaa gguggggaug 1200
   acgucaaauc aucaugcccc uuaugacnug ggcuacacac gugcuacaau ggagaguaca 1260
   acgaguugcg aagucgugag gcuaagcuaa ucucuuaaag cucuucucag uucggauugu 1320
   aggcugcaac ucgccuncau gaagccggaa ucgcuaguaa ucgcggauca gcacgccgcg 1380
   gugaauacgu ucccgggunu nguacacacc gcccgucaca ccangagagu uuguaacacc 1440
   cgaagccggu gggguaaccg cnaggagcca gccgucuaag gugggauaga uga 1493
<210> 23
   <211> 1535
   <212> RNA
   <213> Enterococcus hirae
<400> 23
   ccuggcucag gacgaacgcu ggcggcgugc cuaauacaug caagucgaac gcuucuuuuu     60
   ccaccggagc uugcuccacc ggaaaaagag gaguggcgaa cgggugagua acacgugggu    120
   aaccugccca ucagaagggg auaacacuug gaaacaggug cuaauaccgu auaacaaucg    180
   aaaccgcaug guuuugauuu gaaaggcgcu uucgggugnc gcugauggau ggacccgcgg    240
   ugcauuagcu aguuggugag guaacggcuc accaaggcga cgaugcauag ccgaccugag    300
   agggugaucg gccacauugg gacugagaca cggcccaaac uccuacggga ggcagcagua    360
   gggaaucuuc ggcaauggac gaaagucuga ccgagcaacg ccgcgugagu gaagaagguu    420
   uucggaucgu aaaacucugu uguuagagaa gaacaaggau gagaguaacu guucaucccu    480
   ugacgguauc uaaccagaaa gccacggcua acuacgugcc agcagccgcg guaauacgua    540
   gguggcaagc guuguccgga uuuauugggc guaaagcgag cgcaggcggu uucuuaaguc    600
   ugaugugaaa gcccccggcu caaccgggga gggucauugg aaacugggag acuugagugc    660
   agaagaggag aguggaauuc cauguguagc ggugaaaugc guagauauau ggaggaacac    720
   caguggcgaa ggcggcucuc uggucuguaa cugacgcuga ggcucgaaag cguggggagc    780
   aaacaggauu agauacccug guaguccacg ccguaaacga ugagugcuaa guguuggagg    840
   guuuccgccc uucagugcug cagcuaacgc auuaagcacu ccgccugggg aguacgaccg    900
   caagguugaa acucaaagga auugacgggg gcccgcacaa gcgguggagc augugguuua    960
   auucgaagca acgcgaagaa ccuuaccagg ucuugacauc cuuugaccac ucuagagaua   1020
   gagcuucccc uucgggggca aagugacagg uggugcaugg uugucgucag cucgugucgu   1080
   gagauguugg guuaaguccc gcaacgagcg caacccuuau uguuaguugc caucauuuag   1140
   uugggcacuc uagcaagacu gccggugaca aaccggagga agguggggau gacgucaaau   1200
   caucaugccc cuuaugaccu gggcuacaca cgugcuacaa ugggaaguac aacgagucgc   1260
   aaagucgcga ggcuaagcua aucucuuaaa gcuucucuca guucggauug uaggcugcaa   1320
   cucgccuaca ugaagccgga aucgcuagua aucgcggauc agcacgccgc ggugaauacg   1380
   uucccgggcc uuguacacac cgcccgucac accacgagag uuuguaacac ccgaagucgg   1440
   ugagguaacc uuuuggagcc agccgccuaa ggugggauag augauugggg ugaagucgua   1500
   acaagguagc cguaucggaa ggugcggcug gauca                                1535
<210> 24
   <211> 1435
   <212> DNA
   <213> Tetragenococcus halophilus
<220>
   <221> modified_base
   <222> (6)..(6)
   <223> n is a, c, g, or t
<220>
   <221> modified_base
   <222> (53) .. (53)
   <223> n is a, c, g, or t
<220>
   <221> modified_base
   <222> (253)..(253)
   <223> n is a, c, g, or t
<220>
   <221> modified_base
   <222> (298)..(298)
   <223> n is a, c, g, or t
<220>
   <221> modified_base
   <222> (1094)..(1094)
   <223> n is a, c, g, or t
<220>
   <221> modified_base
   <222> (1182)..(1182)
   <223> n is a, c, g, or t
<220>
   <221> modified_base
   <222> (1426)..(1426)
   <223> n is a, c, g, or t
<400> 24
   aatacntgca agtcgaacgc tgcttaagaa gaaacttcgg ttttttctta agnggagtgg     60
   cggacgggtg agtaacacgt ggggaaccta tccatcagcg ggggataaca cttggaaaca    120
   ggtgctaata ccgcatatgg ctttttttca cctgaaagaa agctcaaagg cgctttacag   180
   cgtcactgat ggctggtccc gcggtgcatt agccagttgg tgaggtaacg gctcaccaaa    240
   gcaacgatgc atngccgacc tgagagggtg atcggccaca ctgggactga gacacggncc    300
   agactcctac gggaggcagc agtagggaat cttcggcaat ggacgcaagt ctgaccgagc    360
   aacgccgcgt gagtgaagaa ggttttcgga tcgtaaagct ctgttgtcag caaagaacag    420
   gagaaagagg aaatgctttt tctatgacgg tagctgacca gaaagccacg gctaactacg    480
   tgccagcagc cgcggtaata cgtaggtggc aagcgttgtc cggatttatt gggcgtaaag 540
   cgagcgcagg cggtgattta agtctgatgt gaaagccccc agctcaactg gggagggtca 600
   ttggaaactg gatcacttga gtgcagaaga ggagagtgga attccatgtg tagcggtgaa 660
   atgcgtagat atatggagga acaccagtgg cgaaggcggc tctctggtct gtaactgacg 720
   ctgaggctcg aaagcgtggg tagcaaacag gattagatac cctggtagtc cacgccgtaa 780
   acgatgagtg ctaagtgttg gagggtttcc gcccttcagt gctgcagtta acgcattaag 840
   cactccgcct ggggagtacg accgcaaggt tgaaactcaa aggaattgac gggggcccgc 900
   acaagcggtg gagcatgtgg tttaattcga agcaacgcga agaaccttac caggtcttga 960
   catcctttga ccgccctaga gatagggttt ccccttcggg ggcaaagtga caggtggtgc 1020
   atggttgtcg tcagctcgtg tcgtgagatg ttgggttaag tcccgtaacg agcgcaaccc 1080
   ttattgttag ttgncagcat tgagttgggc actctagcaa gactgccggt gacaaaccgg 1140
   aggaaggcgg ggatgacgtc aaatcatcat gccccttatg anctgggcta cacacgtgct 1200
   acaatgggaa gtacaacgag caagccaagc cgcaaggcct agcgaatctc tgaaagcttc 1260
   tctcagttcg gattgcaggc tgcaactcgc ctgcatgaag ccggaatcgc tagtaatcgc 1320
   ggatcagcat gccgcggtga atccgttccc gggccttgta cacaccgccc gtcacaccac 1380
   gagagtttgt aacacccaaa gtcggtgcgg caacccttcg gggagncagc cgcct 1435
<210> 25
   <211> 1452
   <212> RNA
   <213> Escherichia coli
<220>
   <221> modified_base
   <222> (491)..(492)
   <223> n is a, c, g, or u
<220>
   <221> modified_base
   <222> (567)..(567)
   <223> n is a, c, g, or u
<220>
   <221> modified_base
   <222> (571) .. (571)
   <223> n is a, c, g, or u
<220>
   <221> modified_base
   <222> (852)..(853)
   <223> n is a, c, g, or u
<400> 25
   aguuugauca uggcucagau ugaacgcugg cggcaggccu aacacaugca agucgaacgg 60
   uaacaggaac gagcuugcug cuuugcugac gaguggcgga cgggugagua augucuggga 120
   aacugccuga uggaggggga uaacuacugg aaacgguagc uaauaccgca uaacgucgca 180
   agaccaaaga gggggaccuu cgggccucuu gccaucggau gugcccagau gggauuagcu 240
   aguagguggg guaaaggcuc accuaggcga cgaucccuag cuggucugag aggaugacca 300
   gccacacugg aacugagaca cgguccagac uccuacggga ggcagcagug gggaauauug 360
   cacaaugggc gcaagccuga ugcagccaug ccgcguguau gaagaaggcc uucggguugu 420
   aaaguacuuu cagcggggag gaagggagua aaguuaauac cuuugcucau ugacguuacc 480
   cgcagaagaa nnaccggcua acuccgugcc agcagccgcg guaauacgga gggugcaagc 540
   guuaaucgga auuacugggc guaaagngca ngcaggcggu uuguuaaguc agaugugaaa 600
   uccccgggcu caaccuggga acugcaucug auacuggcaa gcuugagucu cguagagggg 660
   gguagaauuc cagguguagc ggugaaaugc guagagaucu ggaggaauac cgguggcgaa 720
   ggcggccccc uggacgaaga cugacgcuca ggugcgaaag cguggggagc aaacaggauu 780
   agauacccug guaguccacg ccguaaacga ugucgacuug gagguugugc ccuugaggcg 840
   uggcuuccgg annuaacgcg uuaagucgac cgccugggga guacggccgc aagguuaaaa 900
   cucaaaugaa uugacggggg ccgcacaagc gguggagcau gugguuuaau ucgaugcaac 960
   gcgaagaacc uuaccugguc uugacaucca cggaaguuuu cagagaugag aaugugccuu 1020
   cgggaaccgu gagacaggug cugcauggcu gucgucagcu cguguuguga aauguugggu 1080
   uaagucccgc aacgagcgca acccuuaucc uuuguugcca gcgguccggc cgggaacuca 1140
   aaggagacug ccagugauaa acuggaggaa gguggggaug acgucaaguc aucauggccc 1200
   uuacgaccag ggcuacacac gugcuacaau ggcgcauaca aagagaagcg accucgcgag 1260
   agcaagcgga ccucauaaag ugcgucguag uccggauugg agucugcaac ucgacuccau 1320
   gaagucggaa ucgcuaguaa ucguggauca gaaugccacg gugaauacgu ucccgggccu 1380
   uguacacacc gcccgucaca ccaugggagu ggguugcaaa agaaguaggu agcuuaaccu 1440
   ucgggagggc gc 1452
<210> 26
   <211> 1541
   <212> RNA
   <213> Salmonella bovis
<400> 26
   aaauugaaga guuugaucau ggcucagauu gaacgcuggc ggcaggccua acacaugcaa 60
   gucgaacggu aacaggaaga agcuugcucg cugcugacga guggcggacg ggugcguaau 120
   gucugggaaa cugccugaug gagggggaua acuacuggaa acgguggcua aucccgcaua 180
   acgucgcaag accaaagagg gggaccucca ggccucuucc caucggaugu gcccagaugg 240
   gauuagcuag uuggugaggu aacggcucac caaggcgacg aucccuagcu ggucugagag 300
   gaugaccagc cacacuggaa cugagacacg guccagacuc cuacgggagg cagcaguggg 360
   gaauauugca cagugugcgc aagccugaug cagccaugcc gccuguauga agaaggccuu 420
   cggguuguaa aguacuuuca gcggggagga agguguugug guuaauaacu gcagcaauug 480
   acguuacccg cagaagaagc accggcuaac uccgugccag cagccgcggu aauacggagg 540
   gugcaagcgu uaaucggaau uacugggcgu aaagcgcacg caggcgguuu guuaagucag 600
   augugaaauc cccgggcuca accugggaac ugcaucugau acuggcaagc uugagucucg 660
   uagagggggg uagaauucca gguguagcgg ugaaaugcgu agagaucugg aggaauaccg 720
   guggcgaagg cggcccccug gacgaagacu gacgcucagg ugcgaaagcg uggggagcaa 780
   acaggauuag auacccuggu aguccacgcc guaaacgaug ucuacuugga gguugugccc 840
   uugaggcgug gcuuccggag cuaacgcguu aaguagaccg ccuggggagu acggccgcaa 900
   gguuaaaacu caaaugaauu gacgggggcc cgcacaagcg guggagcaug ugguuuaauu 960
   ccaugcaacu cuaagaaccu uaccugguca ugacauccac agaacuuucc agagaugaga 1020
   cugugccuuc gggaacugug agacaggugc ugcauggcug ucgucagcuc guguugugaa 1080
   auguuggguu aagucccgca acgagcgcaa cccuuauccu uuguugccag cgguccggcc 1140
   gggaacucaa aggagacugc cagugauaaa cuggaggaag guggggauga cgucaaguca 1200
   ucaugccccu uacgaccagg gcuacacacg ugcuacaaug gcgcauacaa agagaagcga 1260
   ccucgcgaga gcaagcggac cucauaaagu gcgucguagu ccggauugga gucugcaacu 1320
   cgacuccaug aagucggaau cgcuaguaau cguggaucag aaugccacgg ugaauacguu 1380
   cccgggccuu guacacaccg cccgucacac caugggagug gguugcaaaa gaaguaggua 1440
   gcuuaaccuu cgggagggcg cuuaccacuu ugugauucau gacuggggug aagucguaac 1500
   aagguaaccg uaggggaacc ugcgguugga ucaccuccuu a 1541
<210> 27
   <211> 1488
   <212> RNA
   <213> Shigella boydii
<400> 27
   uggcucagau ugaacgcugg cggcaggccu aacacaugca agucgaacgg uaacaggaag      60
   cagcuugcug uuucgcugac gaguggcgga cgggugagua augucuggga aacugccuga     120
   uggaggggga uaacuacugg aaacgguagc uaauaccgca uaacgucgca agaccaaaga     180
   gggggaccuu cgggccucuu gccaucggau gugcccagau gggauuagcu uguugguggg    240
   guaacggcuc accaaggcga cgaucccuag cuggucugag aggaugacca gccacacugg     300
   aacugagaca cgguccagac uccuacggga ggcagcagug gggaauauug cacaaugggc     360
   gcaagccuga ugcagccaug ccgcguguau gaagaaggcc uucggguugu aaaguacuuu     420
   cagcggggag gaagggagua aaguuaauac cuuugcucau ugacguuauc cgcagaagaa     480
   gcaccggcua acuccgugcc agcagccgcg guaauacgga gggugcaagc guuaaucgga     540
   auuacugggc guaaagcgca cgcaggcggu uuguuaaguc agaugugaaa uccccgggcu     600
   caaccuggga acugcaucug auacuggcaa gcuugagucu cguagagggg gguagaauuc     660
   cagguguagc ggugaaaugc guagagaucu ggaggaauac cgguggcgaa ggcggccccc     720
   uggacgaaga cugacgcuca ggugcgaaag cguggggagc aaacaggauu agauacccug     780
   guaguccacg ccguaaacga ugucgacuug gagguugugc ccuugaggcg uggcuuccgg     840
   agcuaacgcg uuaagucgac cgccugggga guacggccgc aagguuaaaa cucaaaugaa     900
   uugacggggg cccgcacaag cgguggagca ugugguuuaa uucgaugcaa cgcgaagaac     960
   cuuaccuggu cuugacaucc acggaaguuu ucagagauga gaaugugccu ucgggaaccg    1020
   ugagacaggu gcugcauggc ugucgucagc ucguguugug aaauguuggg uuaagucccg    1080
   caacgagcgc aacccuuauc cuuuguugcc agcgguccgg ccgggaacuc aaaggagacu    1140
   gccagugaua aacuggagga agguggggau gacgucaagu caucauggcc cuuacgacca     1200
   gggcuacaca cgugcuacaa uggcgcauac aaagagaagc gaccucgcga gagcaagcgg    1260
   accucauaaa gugcgucgua guccggauug gagucugcaa cucgacucca ugaagucgga    1320
   aucgcuagua aucguggauc agaaugccac ggugaauacg uucccgggcc uuguacacac    1380
   cgcccgucac accaugggag uggguugcaa aagaaguagg uagcuuaacc uucgggaggg     1440
   cgcuuaccac uuugugauuc augacugggg ugaagucgua acaaggua                 1488
<210> 28
   <211> 1471
   <212> RNA
   <213> Shigella dysenteriae
<220>
   <221> modified_base
   <222> (1)..(2)
   <223> n is a, c, g, or u
<220>
   <221> modified_base
   <222> (1470)..(1470)
   <223> n is a, c, g, or u
<400> 28
   nnauugaaga guuugaucau ggcucagauu gaacgcuggc ggcaggccua acacaugcaa 60
   gucgaacggu aacaggaagc agcuugcugc uuugcugacg aguggcggac gggugaguaa 120
   ugucugggaa acugccugau ggagggggau aacuacugga aacgguagcu aauaccgcau 180
   aacgucgcaa gaccaaagag ggggaccuuc gggccucuug ccaucggaug ugcccagaug 240
   ggauuagcua guaggugggg uaauggcuca ccuaggcgac gaucccuagc uggucugaga 300
   ggaugaccag ccacacugga acugagacac gguccagacu ccuacgggag ggagcagugg 360
   ggaauauugc acaaugggcg caagccugau gcagccaugc cgcguguaug aagaaggcuu 420
   cggguuguaa aguacuuuca gcggggagga agggaguaaa guuaauaccu uugcucauug 480
   acguuacccg cagaagaagc accggcuaac uccgugccag cagccgcggu aauacggagg 540
   gugcaagcgu uaaucggaau uacugggcgu aaagcgcacg caggcgguuu guuaagucag 600
   augugaaauc cccgggcuca accugggaac ugcaucugau acuggcaagc uugagucucg 660
   uagagggggg uagaauucca gguguagcgg ugaaaugcgu agagaucugg aggaauaccg 720
   guggcgaagg cggcccccug gacgaagacu gacgcucagg ugcgaaagcg uggggagcaa 780
   acaggauuag auacccuggu aguccacgcc guaaacgaug ucgacuugga gguugugccc 840
   uugaggcgug gcuuccggag cuaacgcguu aagucgaccg ccuggggagu acggccgcaa 900
   gguuaaaacu caaaugaauu gacgggggcc gcacaagcgg uggagcaugu gguuuaauuc 960
   gaugcaacgc gaagaaccuu accuggucuu gacauccacg gaaguuuuca gagaugagaa 1020
   ugugccuucg ggaaccguga gacaggugcu gcauggcugu cgucagcucg uguugugaaa 1080
   uguuggguua agucccgcaa cgagcgcaac ccuuauccuu uguugccagc gguccggccg 1140
   ggaacucaaa ggagacugcc agugauaaac uggaggaagg uggggaugac gucaagucau 1200
   cauggcccuu acgaccaggg cuacacacgu gcuacaaugg cgcauacaaa gagaagcgac 1260
   cucgcgagag caagcggacc ucauaaagug cgucguaguc cggauuggag ucugcaacuc 1320
   gacuccauga agucggaauc gcuaguaauc guggaucaga augccacggu gaauacguuc 1380
   ccgggccuug uacacaccgc ccgucacacc augggagugg guugcaaaag aaguagguag 1440
   cuuaacuucg ggagggcgcu uaccacuuun u 1471
<210> 29
   <211> 1468
   <212> RNA
   <213> Shigella flexneri
<220>
   <221> modified_base
   <222> (1) .. (2)
   <223> n is a, c, g, or u
<220>
   <221> modified_base
   <222> (372)..(372)
   <223> n is a, c, g, or u
<400> 29
   nnauugaaga guuugaucau ggcucagauu gaacgcuggc ggcaggccua acacaugcaa     60
   gucgaacggu aacaggaagc agcuugcugu uucgcugacg aguggcggac gggugaguaa    120
   ugucugggaa acugccugau ggagggggau aacuacugga aacgguagcu aauaccgcau    180
   aacgucgcaa gaccaaagag ggggaccuuc gggccucuug ccaucggaug ugcccagaug    240
   ggauuagcua guaggugggg uaacggcuca ccuaggcgac gaucccuagc uggucugaga    300
   ggaugaccag ccacacugga acugagacac gguccagacu ccuacgggag gcagcagugg    360
   ggaauauugc anaaugggcg caagccugau gcagccaugc cgcguguaug aagaaggccu    420
   ucggguugua aaguacuuuc agcggggagg aagggaguaa aguuaauacc uuugcucauu    480
   gacguuaccc gcagaagaag caccggcuaa cuccgugcca gcagccgcgg uaauacggag    540
   ggugcaagcg uuaaucggaa uuacugggcg uaaagcgcac gcaggcgguu uguuaaguca    600
   gaugugaaau ccccgggcuc aaccugggaa cugcaucuga uacuggcaag cuugagucuc    660
   guagaggggg guagaauucc agguguagcg gugaaaugcg uagagaucug gaggaauacc    720
   gguggcgaag gcggcccccu ggacgaagac ucacgcucag gugcgaaagc guggggagca    780
   aacaggauua gauacccugg uaguccacgc uguaaacgau gucgacuugg agguugugcc    840
   cuugaggugu ggcuuccgga cguaacgcgu uaagucgacc gccuggggag uacggccgca    900
   agguuaaaac ucaaaugaau ugacgggggc cgcacaagcg guggagcaug ugguuuaauu    960
   cgaugcaacg cgaagaaccu uaccuggucu ugacauccac ggaaguuuuc agagaugaga   1020
   augugccuuc gggaaccgug agacaggugc ugcauggcug ucgucagcuc guguugugaa   1080
   auguuggguu aagucccgca acgagcgcaa cccuuauccu uuguugccag cgguccggcc   1140
   gggaacucaa aggagacugc cagugauaaa cuggaggaag guggggauga cgucaaguca   1200
   ucauggcccu uacgaccagg gcuacacacg ugcuacaaug gcgcauacaa agagaagcga   1260
   ccucgcgaga gcaagcggac cucacaaagu gcgucguagu ccggauugga gucugcaacu   1320
   cgacuccaug aagucggaau cgcuaguaau cguggaucag aaugccacgg ugaauacguu   1380
   cccgggccuu guacacaccg cucgucacac caugggagug gguuguaaaa gaaguaggua   1440
   gcuuaacuuc gggagggcgc uuaccacu                                      1468
<210> 30
   <211> 1541
   <212> RNA
   <213> Streptococcus bovis
<400> 30
   agaguuugau ccuggcucag gacgaacgcu ggcggcgugc cuaauacaug caaguagaac     60
   gcugaagacu uuagcuugcu aaaguuggaa gaguugcgaa cgggugagua acgcguaggu    120
   aaccugccua cuagcggggg auaacuauug gaaacgauag cuaauaccgc auaacagcau    180
   uuaacacaug uuagaugcuu gaaaggagca auugcuucac uaguagaugg accugcguug    240
   uauuagcuag uuggugaggu aacggcucac caaggcgacg auacauagcc gaccugagag    300
   ggugaucggc cacacuggga cugagacacg gcccagacuc cuacgggagg cagcaguagg    360
   gaaucuucgg caaugggggc aacccugacc gagcaacgcc gcgugaguga agaagguuuu    420
   cggaucguaa agcucuguug uaagagaaga acguguguga gaguggaaag uucacacagu    480
   gacgguaacu uaccagaaag ggacggcuaa cuacgugcca gcagccgcgg uaauacguag    540
   gucccgagcg uuguccggau uuauugggcg uaaagcgagc gcaggcgguu uaauaagucu    600
   gaaguuaaag gcaguggcuu aaccauuguu cgcuuuggaa acuguuagac uugagugcag    660
   aaggggagag uggaauucca uguguagcgg ugaaaugcgu agauauaugg aggaacaccg    720
   guggcgaaag cggcucucug gucuguaacu gacgcugagg cucgaaagcg uggggagcaa    780
   acaggauuag auacccuggu aguccacgcc guaaacgaug agugcuaggu guuaggcccu    840
   uuccggggcu uagugccgca gcuaacgcau uaagcacucc gccuggggag uacgaccgca    900
   agguugaaac ucaaaggaau ugacgggggc ccgcacaagc gguggagcau gugguuuaau    960
   ucgaagcaac gcgaagaacc uuaccagguc uugacauccc gaugcuauuc cuagagauag   1020
   gaaguuucuu cggaacaucg gugacaggug gugcaugguu gucgucagcu cgugucguga   1080
   gauguugggu uaagucccgc aacgagcgca accccuauug uuaguugcca ucauuaaguu   1140
   gggcacucua gcgagacugc cgguaauaaa ccggaggaag guggggauga cgucaaauca   1200
   ucaugccccu uaugaccugg gcuacacacg ugcuacaaug guugguacaa cgagucgcga   1260
   gucggugacg gcaagcaaau cucuuaaagc caaucucagu ucggauugua ggcugcaacu   1320
   cgccuacaug aagucggaau cgcuaguaau cgcggaucag cacgccgcgg ugaauacguu   1380
   cccgggccuu guacacaccg cccgucacac cacgagaguu uguaacaccc gaagucggug   1440
   agguaaccuu uuaggagcca gccgccuaag gugggauaga ugauuggggu gaagucguaa   1500
   caagguagcc guaucggaag gugcggcugg aucaccuccu u                        1541
<210> 31
   <211> 1492
   <212> RNA
   <213> Streptococcus infantarius
<400> 31
   gcucaggacu aacgcuggcg gcgugccuaa uacaugcaag uagaacgcug aaaacuuuag 60
   cuugcuaaag uuugaagagu ugcgaacggg ugaguaacgc guagguaacc ugccuacuag 120
   cgggggauaa cuauuggaaa cgauagcuaa uaccgcauaa cagcauuuaa cccauguuag 180
   augcuugaaa ggagcaauug cuucacuagu agauggaccu gcguuguauu agcuaguugg 240
   ugagguaacg gcucaccaag gcgacgauac auagccgacc ugagagggug aucggccaca 300
   cugggacuga gacacggccc agacuccuac gggaggcagc aguagggaau cuucggcaau 360
   gggggcaacc cugaccgagc aacgccgcgu gagugaagaa gguuuucgga ucguaaagcu 420
   cuguuguaag agaagaaugu gugugagagu ggaaaguuca cacagugacg guaacuuacc 480
   agaaagggac ggcuaacuac gugccagcag ccgcgguaau acguaggucc cgagcguugu 540
   ccggauuuau ugggcguaaa gcgagcgcag gcgguuuaau aagucugaag uuaaaggcag 600
   uggcuuaacc auuguucgcu uuggaaacug uuagacuuga gugcagaagg ggagagugga 660
   auuccaugug uagcggugaa augcguagau auauggagga acaccggugg cgaaagcggc 720
   ucucuggucu guaacugacg cugaggcucg aaagcguggg gagcaaacag gauuagauac 780
   ccugguaguc cacgccguaa acgaugagug cuagguguua ggcccuuucc ggggcuuagu 840
   gccgcagcua acgcauuaag cacuccgccu ggggaguacg accgcaaggu ugaaacucaa 900
   aggaauugac gggggcccgc acaagcggug gagcaugugg uuuaauucga agcaacgcga 960
   agaaccuuac caggucuuga caucccgaug cuauuccuag agauaggaag uuucuucgga 1020
   acaucgguga cagguggugc augguugucg ucagcucgug ucgugagaug uuggguuaag 1080
   ucccgcaacg agcgcaaccc cuauuguuag uugccaucau uaaguugggc acucuagcga 1140
   gacugccggu aauaaaccgg aggaaggugg ggaugacguc aaaucaucau gccccuuaug 1200
   accugggcua cacacgugcu acaaugguug guacmacgag ucgcgagucg gugacggcaa 1260
   gcaaaucucu uaaagccaau cucaguucgg auuguaggcu gcaacucgcc uacaugaagu 1320
   cggaaucgcu aguaaucgcg gaucagcacg ccgcggugaa uacguucccg ggccuuguac 1380
   acaccgcccg ucacaccacg agaguuugua acacccgaag ucggugaggu aaccuuuuag 1440
   gagccagccg ccuaaggugg gauagaugau uggggugaag ucguaacaag gu 1492
<210> 32
   <211> 1487
   <212> RNA
   <213> Streptococcus salivarius
<220>
   <221> modified_base
   <222> (939)..(939)
   <223> n is a, c, g, or u
<220>
   <221> modified_base
   <222> (1059)..(1059)
   <223> n is a, c, g, or u
<400> 32
   uuuaaugaga guuugauccu ggcucaggac gaacgcuggc ggcgugccua auacaugcaa      60
   guagaacgcu gaagagagga gcuugcucuu cuuggaugag uugcgaacgg gugaguaacg     120
   cguagguaac cugccuugua gcgggggaua acuauuggaa acgauagcua auaccgcaua     180
   acaauggaug acccauguca uuuauuugaa aggggcaaau gcuccacuac aagauggacc     240
   ugcguuguau uagcuaguag gugagguaac ggcucaccua ggcgacgaua cauagccgac     300
   cugagagggu gaucggccac acugggacug agacacggcc cagacuccua cgggaggcag     360
   caguagggaa ucuucggcaa ugggggcaac ccugaccgag caacgccgcg ugagugaaga     420
   agguuuucgg aucguaaagc ucuguuguaa gucaagaacg agugugagag uggaaaguuc     480
   acacugugac gguagcuuac cagaaaggga cggcuaacua cgugccagca gccgcgguaa     540
   uacguagguc ccgagcguug uccggauuua uugggcguaa agcgagcgca ggcgguuuga     600
   uaagucugaa guuaaaggcu guggcucaac cauaguucgc uuuggaaacu gucaaacuug     660
   agugcagaag gggagagugg aauuccaugu guagcgguga aaugcguaga uauauggagg      720
   aacaccggug gcgaaagcgg cucucugguc uguaacugac gcugaggcuc gaaagcgugg     780
   ggagcgaaca ggauuagaua cccugguagu ccacgccgua aacgaugagu gcuagguguu     840
   ggauccuuuc cgggauucag ugccgcagcu aacgcauuaa gcacuccgcc uggggaguac     900
   gaccgcaagg uugaaacuca aaggaauuga cgggggccng cacaagcggu ggagcaugug     960
   guuuaauucg aagcaacgcg aagaaccuua ccaggucuug acaucccgau gcuauuucua    1020
   gagauagaaa guuacuucgg uacaucggug acagguggng caugguuguc gucagcucgu    1080
   gucgugagau guuggguuaa gucccgcaac gagcgcaacc ccuauuguua guugccauca    1140
   uucaguuggg cacucuagcg agacugccgg uaauaaaccg gaggaaggug gggaugacgu    1200
   caaaucauca ugccccuuau gaccugggcu acacacgugc uacaaugguu gguacaacga    1260
   guugcgaguc ggugacggca agcuaaucuc uuaaagccaa ucucaguucg gauuguaggc    1320
   ugcaacucgc cuacaugaag ucggaaucgc uaguaaucgc ggaucagcac gccgcgguga    1380
   auacguuccc gggccuugua cacaccgccc gucacaccac gagaguuugu aacacccgaa    1440
   gucggugagg uaaccuuuug gagccagccg ccuaaggugg gauagau                  1487
<210> 33
   <211> 1540
   <212> RNA
   <213> Streptococcus thermophilus
<220>
   <221> modified_base
   <222> (130)..(130)
   <223> n is a, c, g, or u
<400> 33
   agaguuugau ccuggcucag gacgaacggu ggcggcgugc cuaauacaug caaguagaac 60
   gcugaagaga ggagcuugcu cuucuuggau gaguugcgaa cgggugagua acgcguaggu 120
   aaccugccun guagcggggg auaacuauug gaaacgauag cuaauaccgc auaacaaugg 180
   augacacaug ucauuuauuu gaaaggggca auugcuccac uacaagaugg accugcguug 240
   uauuagcuag uaggugaggu aauggcuuac cuaggcgacg auacauagcc gaccugagag 300
   ggugaucggc cacacuggga cugagacacg gcccagacuc cuacgggagg cagcaguagg 360
   gaaucuucgg caaugggggc aacccugacc gagcaacgcc gcgugacuga agaagguuuu     420
   cggaucguaa agcucuguug uaagucaaga acggguguga gaguggaaag uucacacagu     480
   gacgguagcu uaccagaaag ggacggcuaa cuacgugcca gcagccgcgg uaauacguag     540
   gucccgagcg uuguccggau uuauugggcg uaaagcgagc gcaggcgguu ugauaagucu 600
   gaaguuaaag gcuguggcuc aaccauaguu cgcuuuggaa acugucaaac uugagugcag 660
   aaggggagag uggaauucca uguguagcgg ugaaaugcgu agauauaugg aggaacaccg 720
   guggcgaaag cggcucucug gucuguaacu gacgcugagg cucgaaagcg uggggagcga 780
   acaggauuag auacccuggu aguccacgcc guaaacgaug agugcuaggu guuggauccu 840
   uuccgggauu cagugccgca gcuaacgcau uaagcacucc gccuggggag uacgaccgga 900
   agguugaaac ucaaaggaau ugacggggcc cgcacaagcg guggagcaug ugguuuaauu 960
   cgaagcaacg cgaagaaccu uaccaccucu ugacaucccg augcuauuuc uagagauaga 1020
   aaguuacuuu gguacaucgg ugacaggugg ugcaugguug ucgucagcuc gugucgugag 1080
   auguuggguu aagucccgca acgagcgcaa ccccuauugu uaguugccau cauucaguug 1140
   ggcacucuag cgagacugcc gguaauaaac cggaggaagg uggggaugac gucaaaucau 1200
   caugccccuu augaccuggg cuacacacgu gcuacaaugg uugguacaac gaguugcgag 1260
   ucggugacgg cgagcuaauc ucuuaaagcc aaucucaguu cggauuguag gcugcaacuc 1320
   gccuacauga agucggaauc gcuaguaauc gcggaucagc acgccgcggu gaauacguuc 1380
   ccgggccuug uacacaccgc ccgucacacc acgagaguuu guaacacccg aagucgguga 1440
   gguaaccuuu uggagccagc cgccuaaggu gggacagaug auugggguga agucguaaca 1500
   agguagccga uccggaaggu gcggcuggau caccuccuuu 1540
<210> 34
   <211> 1332
   <212> RNA
   <213> Fusobacterium alocis
<220>
   <221> modified_base
   <222> (76)..(76)
   <223> n is a, c, g, or u
<220>
   <221> modified_base
   <222> (80) .. (80)
   <223> n is a, c, g, or u
<220>
   <221> modified_base
   <222> (258) .. (258)
   <223> n is a, c, g, or u
<220>
   <221> modified_base
   <222> (261)..(261)
   <223> n is a, c, g, or u
<220>
   <221> modified_base
   <222> (374) .. (374)
   <223> n is a, c, g, or u
<220>
   <221> modified_base
   <222> (812)..(812)
   <223> n is a, c, g, or u
<220>
   <221> modified_base
   <222> (847)..(847)
   <223> n is a, c, g, or u
<220>
   <221> modified_base
   <222> (999)..(999)
   <223> n is a, c, g, or u
<220>
   <221> modified_base
   <222> (1076)..(1076)
   <223> n is a, c, g, or u
<220>
   <221> modified_base
   <222> (1174)..(1174)
   <223> n is a, c, g, or u
<400> 34
   gggugcggga cgggugagua acgcguaaag aacuugccuc acagauaggg acaacauuua      60
   gaaaugaaug cuaaunccun auauuaugaa aauagggcau ccuauaauua ugaaagcuaa     120
   augcgcugug agagagcuuu gcgucccauu agcuaguugg agagguaacg gcucaccaag     180
   gcgaugaugg guagccggcc ugagagggug aacggccaca aggggacuga gacacggccc     240
   uuacuccuac gggaggcngc nguggggaau auuggacaau ggaccgagag ucugauccag     300
   caauucugug ugcacgauga aguuuuucgg aauguaaagu gcuuucaguu gggaagaaaa     360
   gaaugacggu accnacagaa gaagugacgg cuaaauacgu gccagcagcc gcgguaauac     420
   guaugucacg agcguuaucc ggauuuauug ggcguaaagc gcgucuaggu gguuauguaa     480
   gucugaugug aaaaugcagg gcucaacucu guauugcguu ggaaacugug uaacuagagu     540
   acuggagagg uaagcggaac uacaagugua gaggugaaau ucguagauau uuguaggaau     600
   gccgaugggg aagccagcuu acuggacaga uacugacgcu aaagcgcgaa agcgugggua     660
   gcaaacagga uuagauaccc ugguagucca cgccguaaac gaugauuacu agguguuggg     720
   ggucgaaccu cagcgcccaa gcuaacgcga uaaguaaucc gccuggggag uacguacgca     780
   aguaugaaac ucaaaggaau ugacggggac cngcacaagc gguggagcau gugguuuaau     840
   ucgacgnaac gcgaggaacc uuaccagcgu uugacaucuu aggaaugaga uagagauaua     900
   ucagugucuu cggggaaacc uaaagacagg uggugcaugg cugucgucag cucgugucgu     960
   gagauguugg guuaaguccc gcaacgagcg caaccccunu cguauguuac caucauuaag    1020
   uuggggacuc augcgauacu gccugcgaug agcaggagga agguggggau gacgunaagu    1080
   caucaugccc cuuauacgcu gggcuacaca cgugcuacaa uggguagaac agagaguugc     1140
   aaagccguga gguggagcua aucucagaaa acunuucuua guucggauug uacucugcaa    1200
   cucgaguaca ugaaguugga aucgcuagua aucgcgaauc agcaaugucg cggugaauac    1260
   guucucgggu cuuguacaca ccgcccguca caccacgaga guugguugca ccugaaguag    1320
   caggccuaac cg                                                        1332
<210> 35
   <211> 1461
   <212> RNA
   <213> Fusobacterium nucleatum
<220>
   <221> modified_base
   <222> (288)..(288)
   <223> n is a, c, g, or u
<220>
   <221> modified_base
   <222> (637)..(637)
   <223> n is a, c, g, or u
<220>
   <221> modified_base
   <222> (744)..(746)
   <223> n is a, c, g, or u
<220>
   <221> modified_base
   <222> (941)..(941)
   <223> n is a, c, g, or u
<400> 35
   auugaacgaa gaguuugauc cuggcucagg augaacgcug acagaaugcu uaacacaugc     60
   aagucuacuu gaauuugggu uuuuuaacuu cgauuugggu ggcggacggg ugaguaacgc    120
   guaaagaacu ugccucacag cuagggacaa cauuuggaaa cgaaugcuaa uaccuaauau    180
   uaugauuaua gggcauccua gaauuaugaa agcuauaugc gcugugagag agcuuugcgu    240
   cccauuagcu aguuggagag guaacggcuc accaaggcaa ugaugggnag ccggccugag    300
   agggugaacg gccacaaggg gacugagaca cggcccuuac uccuacggga ggcagcagug    360
   gggaauauug gacaauggac cgagagucug auccagcaau ucugugugca cgaugacguu    420
   uuucggaaug uaaagugcuu ucaguuggga agaaaaaaau gacgguacca acagaagaag    480
   ugacggcuaa auacgugcca gcagccgcgg uaauacguau gucacgagcg uuauccggau    540
   uuauugggcg uaaagcgcgu cuaggugguu auguaagucu gaugugaaaa ugcagggcuc    600
   aacucuguau ugcguuggaa acuguguaac uagaguncug gaaagguaag cggaacuaca    660
   aguguagagg ugaaauucgu agauauuugu aggaaugccg auggggaagc cagcuuacug     720
   gacagauacu gacgcugaag cgcnnnagcg uggguagcaa acaggauuag auacccuggu    780
   aguccacgcc guaaacgaug auuacuaggu guuggggguc gaaccucagc gcccaagcaa     840
   acgcgauaag uaauccgccu ggggaguacg uacgcaagua ugaaacucaa aggaauugac    900
   ggggacccgc acaagcggug gagcaugugg uuuaauucga ngcaacgcga ggaaccuuac    960
   cagcguuuga caucuuagga augagacaga gauguuucag ugucccuucg gggaaaccua   1020
   aagacaggug gugcauggcu gucgucagcu cgugucguga gauguugggu uaagucccgc   1080
   aacgagcgca accccuuucg uauguuacca ucauuaaguu ggggacucau gcgauacugc   1140
   cuacgaugag uaggaggaag guggggauga cgucaaguca ucaugccccu uauacgcugg   1200
   gcuacacacg ugcuacaaug gguagaacag agaguugcaa agccgugagg uggagcuaau   1260
   cucagaaaac uauucuuagu ucggauugua cucugcaacu cgaguacaug aaguuggaau   1320
   cgcuaguaau cgcgaaucag caaugucgcg gugaauacgu ucucgggucu uguacacacc   1380
   gcccgucaca ccacgagagu ugguugcacc ugaaguagca ggccuaaccg uaaggaggga   1440
   uguuccgagg gugugauuag c                                             1461
<210> 36
   <211> 1452
   <212> RNA
   <213> Fusobacterium varium
<220>
   <221> modified_base
   <222> (256)..(256)
   <223> n is a, c, g, or u
<220>
   <221> modified_base
   <222> (271).. (271)
   <223> n is a, c, g, or u
<220>
   <221> modified_base
   <222> (320)..(320)
   <223> n is a, c, g, or u
<220>
   <221> modified_base
   <222> (422)..(422)
   <223> n is a, c, g, or u
<220>
   <221> modified_base
   <222> (509)..(509)
   <223> n is a, c, g, or u
<220>
   <221> modified_base
   <222> (683)..(683)
   <223> n is a, c, g, or u
<220>
   <221> modified_base
   <222> (844)..(844)
   <223> n is a, c, g, or u
<220>
   <221> modified_base
   <222> (891)..(891)
   <223> n is a, c, g, or u
<220>
   <221> modified_base
   <222> (923)...(926)
   <223> n is a, c, g, or u
<220>
   <221> modified_base
   <222> (998)..(998)
   <223> n is a, c, g, or u
<220>
   <221> modified_base
   <222> (1157)..(1158)
   <223> n is a, c, g, or u
<220>
   <221> modified_base
   <222> (1447)..(1447)
   <223> n is a, c, g, or u
<400> 36
   aacgaagagu uugauccugg cucaggauga acgcugacag aaugcuuaac acaugcaagu 60
   cuacuugauc uucgggugaa gguggcggac gggugaguaa cgcguaaaga acuugccuua 120
   cagacuggga caacauuugg aaacgaaugc uaauaccgga uauuaugacu gagucgcaug 180
   auuugguuau gaaagcuaua ugcgcuguga gagagcuuug cgucccauua guuaguuggu 240
   gagguaacgg cucacnaaga cgaugauggg nagccggccu gagaggguga acggccacaa 300
   ggggacugag acacggcccn yacuccuacg ggaggcagca guggggaaua uuggacaaug 360
   gaccaaaagu cugauccagc aauucugugu gcacgaugaa guuuuucgga auguaaagug 420
   cnuucaguug ggaagaaguc agugacggua ccaacagaag aagcgacggc uaaauacgug 480
   ccagcagccg cgguaauacg uaugucgcna gcguuauccg gauuuauugg gcguaaagcg 540
   cgucuaggcg guuuaguaag ucugauguga aaaugcgggg cucaaccccg uauugcguug 600
   gaaacugcua aacuagagua cuggagaggu aggcggaacu acaaguguag aggugaaauu 660
   cguagauauu uguaggaaug ccnaugggga agccagccua cuggacagau acugacgcua 720
   aagcgcgaaa gcguggguag caaacaggau uagauacccu gguaguccac gccguaaacg 780
   augauuacua gguguugggg gucgaaccuc agcgcccaag cuaacgcgau aaguaauccg 840
   ccungggagu acguacgcaa guaugaaacu caaaggaauu gacggggacc ngcacaagcg 900
   guggagcaug ugguuuaauu cgnnnnaacg cgaggaaccu uaccagcguu ugacauccca 960
   agaaguuaac agagauguuu ucgugccucu ucggaggnac uuggugacag guggugcaug 1020
   gcugucguca gcucgugucg ugagauguug gguuaagucc cgcaacgagc gcaaccccuu 1080
   ucguauguua ccaucauuaa guuggggacu caugcgagac ugccugcgau gagcaggagg 1140
   aaggugggga ugacgunnag ucaucaugcc ccuuauacgc ugggcuacac acgugcuaca 1200
   auggguagua cagagagcug caaaccugcg aggguaagcu aaucucauaa aacuauucuu 1260
   aguucggauu guacucugca acucgaguac augaaguugg aaucgcuagu aaucgcaaau 1320
   cagcuauguu gcggugaaua cguucucggg ucuuguacac accgcccguc acaccacgag 1380
   aguugguugc accugaagua acaggccuaa ccguaaggag ggauguuccg agggugugau 1440
   uagcganugg gg 1452
<210> 37
   <211> 20
   <212> DNA
   <213> Artificial Sequence
<220>
   <223> Description of Artificial Sequence: Synthetic oligonucleotide
<400> 37
   agagtttgat cctggctcag 20
<210> 38
   <211> 18
   <212> DNA
   <213> Artificial Sequence
<220>
   <223> Description of Artificial Sequence: Synthetic oligonucleotide
<400> 38
   gctgcctccc gtaggagt

## Description

This application claims the benefit of U.S. Provisional Application No. 60/623,771, filed November 1, 2004 and U.S. Provisional Application No. 60/646,592, filed January 26, 2005.

### BACKGROUND OF THE INVENTION

Ulcerative Colitis and Crohn's disease are chronic inflammatory diseases of the colon and rectum. Although corticosteroids, aminosalicylates, and immunomodulators have provided some benefit in treatment of ulcerative colitis, restorative proctocolectomy ileal-pouch anal anastamosis (RP/IPAA) remains the gold standard for management of chronically active and steroid-refractory disease. The most common and debilitating complication of IPAA is symptomatic inflammation of the ileal reservoir, or pouchitis. Prior studies have demonstrated a significant decrease in quality of life (IBDQ and SF-36) when RP/IPAA is complicated by pouchitis. The incidence of pouchitis is between 30-50% up to 5 years postoperatively, with the majority of initial cases in the first 3-6 months. Clinically, pouchitis is characterized by increased stool frequency, fecal urgency, rectal bleeding, and malaise. However, the diagnosis of pouchitis is a combination of specific clinical, endoscopic, and histologic criteria. There is much debate as to whether pouchitis is an extension of the Ulcerative Colitis or a distinct disease entity. There has been no data to strongly favor either. Although many theories have been proposed, the precise mechanism of disease in pouchitis remains elusive. The dramatic clinical response to antibiotics in pouchitis suggests that microflora may play a causal role. Despite an 80% initial response to antibiotics, 60% of patients have recurring episodes of pouchitis and up to 30% of patients develop chronic symptomatic pouchitis. There have been no studies to date identifying any specific microfloral pattern or organism in the pathogenesis of pouchitis. In this study, we introduce Amplicon Length Heterogeneity, a novel culture-independent technique for detailed microfloral characterization in pouchitis.

WO03053220 describes a method for diagnosing Crohn's disease or ulcerative colitis by using a probe which detects flagellin of Heliobacter bilis or an unknown bacterium wherein an increase indicates a disease status.

Changes in microbial flora have been associated withinflammatory bowel disease and specifically with Crohn's disease and ulcerative colitis (Seksik et al., 2003, Gut 52:237-242; Ott et al., 2004, Gut 53: 685-693; Kleesen at al., Scand J Gsatroenerol 37: 1034-1041; Linskens et al., 2002, Scand J Gastroenterol 36 Suppl 234: 29-40).

### DESCRIPTION OF THE DRAWING

Fig. 1. Histogram of Crohns tissue and lumen ALH Fingerprints.
Fig. 2. Histogram of Ulcerative Colitis tissue and lumen ALH Fingerprints.
Fig. 3. Principal coordinate analysis (PCO) of Crohns and Ulcerative Colitis ALH Fingerprints.
Fig. 4. Histogram of normal Pouch and Pouchitis tissue ALH Fingerprints.
Fig. 5. Principle coordinate analysis (PCO) of Pouchitis ALH Fingerprints.
Fig. 6. Identification of peaks in normal Pouch and Pouchitis Histogram.

### DESCRIPTION OF THE INVENTION

The present description relates to methods and compositions for diagnosing, monitoring, prognosticating, analyzing, etc., polymicrobial diseases. A polymicrobial disease is a disease or condition that is associated with the presence of at least two different microbes, including, e.g., associations between bacteria-bacteria, virus-virus, parasite-parasite, bacteria-virus, bacteria-parasite, and virus-parasite. A preferred method of determining the microbial community present in a polymicrobial disease is amplicon lengh heterogeneity ("ALH").

Examples of polymicrobial diseases include, but are not limited to, e.g., co-infection of Borrelia and Ehrlichia in Lyme borreliosis; mixed viral-bacterial infections during influenza pandemics; respiratory diseases; gastroenteritis; conjunctivitis; keratitis; hepatitis; periodontal diseases; genital infections; intra-abdominal infections; inflammatory bowel diseases; urinary tract infections; necrotizing soft-tissue infection.

The present invention also relates to the microbial community present in the digestive tract and lumen in normal subjects, and subjects with digestive tract diseases, especially diseases of the colon, such as inflammatory bowel disease, including ulcerative colitis, Crohn's syndrome, and pouchitis. The present invention especially relates to compositions and methods for diagnosing, prognosticating, and/or monitoring the disease progression of the mentioned diseases and conditions, e.g., to determine the presence of the disease in a subject, to determine a therapeutic regimen, to determine the onset of active disease, to determine the predisposition to the disease, to determine the course of the disease, etc.

The present invention provides methods for diagnosing and monitoring the disease progression of inflammatory bowel diseases, such as ulcerative colitis, Crohn's disease, or pouchitis, comprising determining the presence or absence of microbes, such as bacteria, in a colon or lumen sample obtained from said subject. The invention is not limited to how the determination is carried out; any suitable method can be used. The term "microbe" includes viruses, bacteria, fungi, and protists. Although the disclosure below may be written in terms of bacteria, any microbe can be used. The invention relates to a method for diagnosing an inflammatory bowel disease in a subject, comprising determining the increase or decrease compared to a normal mucosa sample of *Morexella sp.* of the Pseudomonas group, *Comamonas sp.* of the Acidovorax Group, *Cryseobacterium sp.* of the Cytophaga Group I, *Enterococus sp.* of the Enterococcus Group, *Bacteroides sp.* of the Bacteroides Group, *Propionibacterium sp.* of the Propionibacterium Group, *Ruminoccocus sp.* of the Clostridium Coccoides Group and *Bacteroides sp.* in a colonic mucosal tissue sample from said subject, wherein (a) the inflammatory bowel disease is Crohn's disease and wherein an increase of *Moraxella sp., Comomonas sp.* and *Chryseobacter sp.* and a decrease of *Bacteroides sp., Propionibacterium sp., Enterococcus sp.* and *Ruminococcus sp.* indicates Crohn's disease in said subject, or wherein (b) the inflammatory bowel disease is ulcerative colitis and wherein an increase of *Moraxella sp., Comomonas sp.* and *Enterococcus sp.* and a decrease of *Bacteroides sp., Propionibacterium sp., Chryseobacter sp.* and *Ruminococcus sp.* indicates ulcerative colitis in said subject. The method of the invention may additionally comprise determining the increase or decrease of one or more bacteria selected from the group consisting *of Bacteriodes sp., Ruminococcus sp., Moraxella sp.* and *Comomonas sp.* in a lumen sample of the subject. The invention also relates to a method for diagnosing pouchitis in an ileal-pouch patient, comprising detecting *Fusobacter sp., Propionibacterium sp.* and at least one of the bacteria of the group of *E.coli* and/or *Shigella sp.* in a colonic mucosal tissue sample from said patient, wherein an increase of *Fusobacter sp.* and a decrease of one or more of the bacteria of the group of *E. coli* and/or *Shigella sp.* as compared to a normal pouch sample and the absence of *Propionibacterium sp.* is diagnostic of pouchitis.

The present description relates to any composition or method which is suitable for detecting a microbial community in a sample (e.g., from a subject having a polymicrobial disease), such as a digestive tract, lumen, or stool sample. A lumen sample is from interior of the intestine.

Any marker which is suitable for identifying and distinguishing a microbial type can be utilized in accordance with present invention. These methods can involve detection of nucleic acid (e.g., DNA, RNA, mRNA, tRNA, rRNA, etc), protein (e.g., using antibodies, protein binding reagents), and any other bio-molecule (e.g., lipid, carbohydrates, etc) that is useful for specifically determining the presence or absence of bacteria in a sample. Any variable indicator or non-coding segment (e.g., repetitive elements, etc.) can also be used, as well as indicator genes. ITS regions can be utilized in fungi.

Standard culture methods can also be utilized, where bacteria and other microorganisms are identified by culturing them on a media, e.g., using a selective media (e.g., comprising a bacteria-specific carbon source) and/or where microorganisms are identified by their growth characteristics, morphology, and other criteria typic ally used to determine cell identity and phylogenetic classification. Any of these methods can also be used in combination with cytological and histological methods, where biopsy samples or cultured samples can be stained and visualized (e.g., by sectioning, or by mounting on a slide or other carrier).

As mentioned, the compositions and methods are useful for diagnostic and prognostic purposes associated with polymicrobial diseases, such as inflammatory bowel diseases, including ulcerative colitis, Crohn's disease, and pouchitis. The markers and fingerprints can be utilized to diagnose the diseases, and distinguish them from other diseases of the digestive tract. They can also be used for assessing disease status, severity, and prognosis, alone, or in combination with other tests. For example, the markers can be used in conjunction with the Crohn's disease activity index (CDAI) or the criteria of Trulove and Witts for assessing disease activity in ulcerative colitis. The information about microorganismal status can also be used to determine when to initiate drug treatment or other therapeutic regimens.

The methods and compositions can also be used to monitor the course of the disease in a subject under treatment or monitor the progression of the disease, irrespective of the treatment regimen. For example, patients with inflammatory bowel syndromes may show spontaneous or drug-induced remissions. To monitor the course of the remission and determine when the disease is active, samples can be obtained periodically, and assayed to determine the appearance of the particular microbial markers or fingerprints in the intestinal tissue, lumen, colonic wash, mucosal samples, or stool.

Assessment of the microbial community can be performed on any sample obtained from a subject, including from lumen, colonic wash, intestinal tissue, intestinal mucosa, gastric tissue, gastric mucosa, stool, etc. Samples can be obtained from any part of the digestive tract, especially the small and large intestines. The large intestine or colon is the part of the intestine from the cecum to the rectum. It is divided into eight sections: the cecum, the appendix, the ascending colon, the transverse colon, the descending colon, the sigmoid colon, the rectum, and the anus. A colonic wash is the fluid left in the intestine after a subject has been given a laxative. The intestinal mucosa is the surface lining of the intestinal tract. Subjects include, e.g., animals, humans, non-human primates, mammals, monkeys, livestock, sheep, goats, pigs, pets (e.g., dogs, cats), small animals, reptiles, birds, etc.

Any suitable method can be utilized to obtain samples from the intestine. Endoscopic biopsy is common method in which a fiber optic endoscope is inserted into the gastrointestinal tract through a natural body orifice. The lining of the intestine is directly visualized and a sample is pinched off with forceps attached to a long cable that runs inside the endoscope. Suitable endoscope and instruments for removing biopsy samples are well known, and include those disolosed in, e.g., U.S. Pat. Nos. 6,632,182, and 6,443,909.

Table 3 summarizes bacteria which have been detected in mucosa tissue and lumen from control subjects, and subjects having Crohn's disease or ulcerative. Table 5 summarizes bacteria which have been detected in the mucosa and lumen of subjects having pouchitis and pouchitis control (subjects with restorative procto colectomy, but without post-operative complications). PCR amplicons were cloned and sequenced from these samples. Briefly, DNA was extracted from each pooled sample. The pooled DNA from mucosa comprised DNA from mucosal and other gastrointestinal cells, as well as the bacteria. The first two variable regions of the 16S ribosomal RNA were amplified using universal Eubacterial primers. Subsequently, the amplification mixture was separated and characterized on a fingerprinting gel. The resulting picture of the gel or tabular compilation of the data (see, e.g., Tables 1, 2, and 4) - comprising discrete, individual bands (PCR amplicons) - can be referred to as the "ALH fingerprint." The ALH fingerprint can be further characterized by identifying the length of the individual replicons that comprise it and/or their specific nucleotides sequences. Amplicons from the microbial community can then be cloned and sequenced, where the sequence is correlated with a particular bacterial group, species, or strain. Using this method, the abundance of the clones from each species is proportional to their abundance in the corresponding community, and can be correlated to peaks in the ALH fingerprint. The sequence data can be used to search the Ribosomal database (RDP) using a standard sequence search tool (Megablast) available from the National Center for Biotechnology Information (NCBI) at NIH. See, e.g., Cole JR, Chai B, Marsh TL, Farris RJ, Wang Q, Kulam SA, Chandra S, McGarrell DM, Schmidt TM, Garrity GM, Tiedje JM. The Ribosomal Database Project (RDP-II): previewing a new autoaligner that allows regular updates and the new prokaryotic taxonomy. Nucleic Acids Res 2003 Jan 1;31(1):442-3. The RDP number obtained from the search results can be parsed using a custom PERL script to classify the Division, Subdivision, Group, and Subgroup of each clone, and the results can be tabulated, and imported into EXCEL or other suitable databases.

### Nucleic acid detection methods

Detection methods have a variety of applications, including for diagnostic, prognostic, forensic, and research applications. To accomplish nucleic acid detection, a polynucleotide in accordance with the present invention can be used as a "probe." The term "probe" or "polynucleotide probe" has its customary meaning in the art, e.g., a polynucleotide which is effective to identify (e.g., by hybridization), when used in an appropriate process, the presence of a target polynucleotide to which it is designed. Identification can involve simply determining presence or absence, or it can be quantitative, e.g., in assessing amounts of a polynucleotide (e.g., copies of a ribosomal RNA) present in a sample. As explained in more detail below, any suitable method can be used, including, but limited to, ALH, PCR, nucleotide sequencing, Southern blot, and or DNA microarrays (e.g., where a microarray comprises a plurality of sequences specific for one or more bacteria of the present invention).

Assays can be utilized which permit quantification and/or presence/absence detection of a target nucleic acid in a sample. Assays can be performed at the single-cell level, or in a sample comprising many cells, where the assay is "averaging" expression over the entire collection of cells and tissue present in the sample. Any suitable assay format can be used, including, but not limited to, e.g., Southern blot analysis, Northern blot analysis, polymerase chain reaction ("PCR") (e.g., Saiki et al., Science, 241:53, 1988; U.S. Pat. Nos. 4,683,195, 4,683,202, and 6,040,166; PCR Protocols: A Guide to Methods and Applications, Innis et al., eds., Academic Press, New York, 1990), reverse transcriptase polymerase chain reaction ("RT-PCR"), anchored PCR, rapid amplification of cDNA ends ("RACE") (e.g., Schaefer in Gene Cloning and Analysis: Current Innovations, Pages 99-115, 1997), ligase chain reaction ("LCR") (EP 320 308), one-sided PCR (Ohara et al., Proc. Natl. Acad. Sci., 86:5673-5677, 1989), indexing methods (e.g., U.S. Pat. No. 5,508,169), *in situ* hybridization, differential display (e.g., Liang et al., Nucl. Acid. Res., 21:3269-3275, 1993; U.S. Pat. Nos. 5,262,311, 5,599,672 and 5,965,409; W097/18454; Prashar and Weissman, Proc. Natl. Acad. Sci., 93:659-663, and U.S. Pat. Nos. 6,010,850 and 5,712,126; Welsh et al., Nucleic Acid Res., 20:4965-4970, 1992, and U.S. Pat. No. 5,487,985) and other RNA fingerprinting techniques, nucleic acid sequence based amplification ("NASBA") and other transcription based amplification systems (e.g., U.S. Pat. Nos. 5,409,818 and 5,554,527; WO 88/10315), polynucleotide arrays (e.g., U.S. Pat. Nos. 5,143,854, 5,424,186; 5,700,637, 5,874,219, and 6,054,270; PCT WO 92/10092; PCT WO 90/15070), Qbeta Replicase (PCT/US87/00880), Strand Displacement Amplification ("SDA"), Repair Chain Reaction ("PCR"), nuclease protection assays, subtraction-based methods, Rapid-Scan™, etc. Additional useful methods include, but are not limited to, e.g., template-based amplification methods, competitive PCR (e.g., U.S. Pat. No. 5,747,251), redox-based assays (e.g., U.S. Pat. No. 5,871,918), Taqman-based assays (e.g., Holland et al., Proc. Natl. Acad, Sci., 88:7276-7280, 1991; U.S. Pat. Nos. 5,210,015 and 5,994,063), real-time fluorescence-based monitoring (e.g., U.S. Pat. 5,928,907), molecular energy transfer labels (e.g., U.S. Pat. Nos. 5,348,853, 5,532,129, 5,565,322, 6,030,787, and 6,117,635; Tyagi and Kramer, Nature Biotech., 14:303-309, 1996). Any method suitable for single cell analysis of polynucleotide or protein expression can be used, including *in situ* hybridization, immunocytochemistry, MACS, FACS, flow cytometry, etc. For single cell assays, expression products can be measured using antibodies, PCR, or other types of nucleic acid amplification (e.g., Brady et al., Methods Mol. & Cell. Biol. 2, 17-25, 1990; Eberwine et al., 1992, Proc. Natl. Acad. Sci., 89, 3010-3014, 1992; U.S. Pat. No. 5,723,290). These and other methods can be carried out conventionally, e.g., as described in the mentioned publications.

Many of such methods may require that the polynucleotide is labeled, or comprises a particular nucleotide type useful for detection. The present invention includes such modified polynucleotides that are necessary to carry out such methods. Thus, polynucleotides can be DNA, RNA, DNA:RNA hybrids, PNA, etc., and can comprise any modification or substituent which is effective to achieve detection.

The present invention provides methods for diagnosing or prognosticating ulcerative colitis, Crohn's disease, or pouchitis, or in a subject, comprising, one or more of the following steps in any effective order, e.g., contacting a gastrointestinal tissue or lumen sample comprising nucleic acid with a polynucleotide probe which is specific for at least one bacteria under conditions effective for said probe to hybridize specifically with said nucleic acid, and detecting hybridization between said probe and said nucleic acid, wherein the presence of one or more bacteria selected from the following group said bacteria indicates the disease presence or the disease status of ulcerative colitis, Crohn's disease, or Pouchitis. The method can further comprise obtaining a colon sample, e.g., by endoscopic biopsy, and/or extracting the nucleic acid from the sample.

The phrases "specific for" or "specific to" a microbe has a functional meaning that indicates the probe (or antibody if it used in a protein context) can be used to identify the presence of the target microbe in a sample and distinguish it from non-target microbe. It is also specific in the sense that it can be used to detect target microbe above background noise ("non-specific binding"). This same definition is also applicable to a polynucleotide or antibody probe. Probes can also be described as being specific for a sequence, where a specific sequence is a defined order of nucleotides (or amino acid sequences, if it is a polypeptide sequence) that occurs in the polynucleotide.

The phrase "hybridize specifically" indicates that the hybridization between single-stranded polynucleotides is based on nucleotide sequence complementarity. The effective conditions are selected such that the probe hybridizes to a pre-selected and/or definite target nucleic acid in the sample. For instance, if detection of a polynucleotide for a ribosomal RNA is desired, a probe can be selected which can hybridize to the target ribosomal RNA under high stringent conditions, without significant hybridization to other non-target sequences in the sample. For example, the conditions can be selected routinely which require 100% or complete complementarity between the target and probe.

Contacting the sample with probe can be carried out by any effective means in any effective environment. It can be accomplished in a solid, liquid, frozen, gaseous, amorphous, solidified, coagulated, colloid, etc., mixtures thereof, matrix. For instance, a probe in an aqueous medium can be contacted with a sample which is also in an aqueous medium, or which is affixed to a solid matrix, or vice-versa.

Generally, as used throughout the specification, the term "effective conditions" means, e.g., the particular milieu in which the desired effect is achieved, such as hybridization between a probe and its target, or antibody binding to a target protein. Such a milieu, includes, e.g., appropriate buffers, oxidizing agents, reducing agents, pH, co-factors, temperature, ion concentrations, suitable age and/or stage of cell (such as, in particular part of the cell cycle, or at a particular stage where particular genes are being expressed) where cells are being used, culture conditions (including substrate, oxygen, carbon dioxide, etc.). When hybridization is the chosen means of achieving detection, the probe and sample can be combined such that the resulting conditions are functional for said probe to hybridize specifically to nucleic acid in said sample.

For detecting the presence of a probe specifically hybridized to a target, any suitable method can be used. For example, polynucleotides can be labeled using radioactive tracers such as ³²P, ³⁵S, ³H, or ¹⁴C, to mention some commonly used tracers. Non-radioactive labeling can also be used, e.g., biotin, avidin, digoxigenin, antigens, enzymes, or substances having detectable physical properties, such as fluorescence or the emission or absorption of light at a desired wavelength, etc.

Any test sample in which it is desired to identify the presence or absence of bacteria can be used, including, e.g., blood, urine, saliva, lumen (for extracting nucleic acid, see, e.g., U.S. Pat. No. 6,177,251), stool, swabs comprising tissue, biopsied tissue, tissue sections, cultured cells, intestinal wash, colonic wash, intestinal mucosa, etc

The results for any of the assays mentioned herein (including the assays in other sections below) can be with respect to a control sample. For example, an increase or decrease can be with respect (in comparison) to a normal lumen or mucosa sample. The normal sample can be from the same patient, but from an unafflicted region or period (e.g., when the patient is in remission). It can also be from a standard value that is calculated based on a normalized population of individuals. Standard statistics can be utilized to determine whether the values are significant.

The present description also provides methods for nucleic acid fingerprinting the community of microbes present in a sample, e.g., using universal primers to the microorganisms in question, whether they be Eubacteria, Archaebacteria, Fungi, or Protists. Since each sample contains a distinctive population of microbes that is representative of the disease, sampling the nucleic acids from the microbes can produce a distinctive array of polynucleotide fragments associated with the disease. These can be presented by any physical characteristic, including size, sequence, mobility, molecular weight (e.g., using mass spectroscopy), etc. Any fingerprinting method can be used, including, e.g., AFLP, ALH, LH-PCR, ARISA, RAPD, etc. Tables 1, 2, and 4 show the frequency of amplicons in various control and disease samples. Although one particular amplicons may not be diagnostic of the condition 100% of the time, using multiple amplicons increases the diagnostic certainty. Moreover, when a condition is being monitored, it may be advantageous to monitor a complex fingerprint (such as shown in Table 1) a it differs from one sampling time to another.

Along these lines, the present description provides method for diagnosing, prognosticating, or monitoring the disease progression of a polymicrobial disease (e.g., an inflammatory bowel disease, such as ulcerative colitis, pouchitis, or Crohn's disease), comprising one or more of the following steps in any effective order, e.g., performing an amplification reaction on a sample comprising nucleic acid with at least two polynucleotide probe primers which are effective for amplifying the microbial community present in said sample, and detecting the reaction products of said amplification reaction, whereby said reaction products comprise a pattern that indicate the presence of the disease or the disease status.

By "disease status," it is meant the relative condition of the disease as compared to its condition at a previous time. For example, when sample reaction products differ (e.g., in quantity or size) from a period of disease severity, this would indicate that the disease status of the subject had changed. The reaction products may show a difference before the subject actually manifests symptoms of the disease, and therefore can be used prognostically to predict a relapse. Similarly, a change in the reaction products can also indicate that the disease is improving and/or responding to a treatment regime.

The term "amplification" indicates that the nucleic acid sequences are increased in copy number to an amount or quantity at which they can be detected. Amplification can be carried out conventionally, using any suitable technique, including polymerase chain reaction (PCR), NASBA (e.g., using T7 RNA polymerase), LCR (ligation chain reaction), LH-PCR, ARISA.

Total nucleic can be extracted from a sample, or the sample can be treated in such a way to preferentially extract nucleic acid only from the microbes that are present in it. DNA extractions can be performed with commercially available kits, such as the Bio101 kit from Qbiogene, Inc, Montreal, Quebec. To prevent contamination by multiple samples during the homogenization process of a sample, each individual sample can be processed separately and completely leading to high yield DNA extractions.

In certain embodiments of the present invention, ribosomal RNA ("rRNA") can be used to distinguish and detect bacteria. For example, bacterial ribosomes are comprised of a small and large subunit, each which is further comprised of ribosomal RNAs and proteins. The rRNA from the small subunit can be referred to as SSU rRNA, and from the larger subunit as LSU rRNA. A large number of rRNAs have been sequenced, and these are publicly available in various accessible databases. See, e.g., Wuyts et al., The European database on small subunit ribosomal RNA, Nucleic Acids Res., 30, 183-185, 2002; Cole et al., The Ribosomal Database Project (RDP-II): previewing a new autoaligner that allows regular updates and the new prokaryotic taxonomy. Nucleic Acids Res., 31(1): 442-3, 2003. See, also, http://rdp.cme.msu.edu/html/ accessed on June 14, 2004. Any rRNA can be used as a marker, including, but not limited to, 16S, 23S, and 5S.

Primer sequences to rRNA can be designed routinely to detect specific species of bacteria, or to detect groups of bacteria, e.g., where a conserved sequence is characteristic of a bacterial group. ALH-PCR can be accomplished routinely, e.g., using a fluorescently labeled forward primer 27F (5'-[6FAM] AGAGTTTGATCCTGGCTCAG-3') (SEQ ID NO:37) and unlabeled reverse primer 338R' (5'-GCTGCCTCCCGTAGGAGT-3') (SEQ ID NO:38). Both primers are highly specific for Eubacteria (Lane, D.J., 16S/23S rRNA Sequencing, in Nucleic Acid Techniques in Bacterial Systematics, E.S.a.M. Goodfellow, Editor. 1991, John Wiley & Sons Ltd: West Sussex, England. p. 115-175).

Primers can also be utilized which amplify the corresponding region in Archae (Burggraf, S., T. Mayer, R. Amann, S. Schadhauser, C.R. Woese and K.O. Stetter, Identifying Members of the Domain Archaea with rRNA-Targeted Oligonicleotide Probes. App. Environ. Microbiol., 1994. 60: p. 3112-3119), Eukaryotes (Rowan, R. and D.A. Powers, Ribosomal RNA sequences and the diversity of symbiotic dinoflagellates (zooxanthellae). Proceedings of the National Academy of Sciences, USA, 1992. 89: p. 3639-3643), and Fungi (Bomeman, J. and J. Hartin, PCR primers that amplify fungal rRNA genes from Environmental Samples. App. Environ. Microbiol., 2000. 66(10): p. 4356-4360).

Primers can be selected from any nucleic acid of the infectious agent, including from rRNA, tRNA, genomic DNA, etc. The primers can be to variable regions, helices, conserved regions, etc.

Selected primers can be utilized in amplicon length heterogeneity ("ALH") to generate fingerprints that characterize the bacterial community (Ritchie, N.J., et al., Use of Length Heterogeneity PCR and Fatty Acid Methyl Ester Profiles to Characterize Microbial Communities in Soil, Applied and Environmental Microbiology, 2000. 66(4): p. 1668-1675; Suzuki, M., M.S. Rappe, and S.J. Giovannoni, Kinetic bias in estimates of coastal picoplankton community structure obtained by measurements of small-subunit rRNA gene PCR amplicon length heterogeneity. Applied and Environmental Microbiology [Appl. Environ. Microbiol.]. 1998. 64(11): p. 4522-4529; Litchfield, C.D. and P.M. Gillevet, Microbial diversity and complexity in hypersaline environments: A preliminary assessment. Journal of Industrial Microbiology & Biotechnology [J. Ind. Microbiol. Biotechnol.]. 2002. 28(1): p. 48-55; Mills, D.K., et al., A Comparison of DNA Profiling Techniques for Monitoring Nutrient Impact on Microbial Community Composition during Bioremediation of Petroleum Contaminated Soils. J. Microbiol. Method, 2003. 54: p. 57-74).

Individual primers can be utilized or a mixture, e.g., comprising degenerate sequences, sequences from one or more group, multiplex reaction where different groups are assessed using primers labeled with different fluorescent tags etc.

The reaction products (i.e., the fragments which are detected after the amplification reaction) can be analyzed by statistical analysis, such as PCO analysis (see Examples) to determine which products are diagnostic of the disease.

### Polypeptide detection

The present description also provides compositions and methods for detecting polypeptides and other biomolecules that are characteristic of the microbial population. For example, the present description provides methods for diagnosing or prognosticating ulcerative colitis, pouchitis, or Crohn's disease comprising:, one or more of the following steps in any effective order, e.g., contacting a sample comprising protein with an antibody which is specific for a bacteria under conditions effective for said antibody to specifically bind to said bacteria, and detecting binding between said antibody and said bacteria.

Polypeptides can be detected, visualized, determined, quantitated, etc. according to any effective method. Useful methods include, e.g., but are not limited to, immunoassays, RIA (radioimmunassay), ELISA, (enzyme-linked-immunosorbent assay), immunoflourescence, flow cytometry, histology, electron microscopy, light microscopy, in situ assays, immunoprecipitation, Western blot, etc.

Immunoassays may be carried in liquid or on biological support. For instance, a sample (e.g., blood, lumen, urine, cells, tissue,cerebral spinal fluid, body fluids, etc.) can be brought in contact with and immobilized onto a solid phase support or carrier such as nitrocellulose, or other solid support that is capable of immobilizing cells, cell particles or soluble proteins. The support may then be washed with suitable buffers followed by treatment with the detectably labeled bacteria specific antibody. The solid phase support can then be washed with a buffer a second time to remove unbound antibody. The amount of bound label on solid support may then be detected by conventional means.

A "solid phase support or carrier" includes any support capable of binding an antigen, antibody, or other specific binding partner. Supports or carriers include glass, polystyrene, polypropylene, polyethylene, dextran, nylon, amylases, natural and modified celluloses, polyacrylamides, and magnetite. A support material can have any structural or physical configuration. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads

One of the many ways in which a bacteria specific antibody can be detectably labeled is by linking it to an enzyme and using it in an enzyme immunoassay (EIA). See, e.g., Voller, A., "The Enzyme Linked Immunosorbent Assay (ELISA)," 1978, Diagnostic Horizons 2, 1-7, Microbiological Associates Quarterly Publication, Walkersville, Md.); Voller, A. et al., 1978, J. Clin. Pathol. 31, 507-520; Butler, J. E., 1981, Meth. Enzymol. 73,482-523; Maggio, E. (ed.), 1980, Enzyme Immunoassay, CRC Press, Boca Raton, Fla.. The enzyme which is bound to the antibody will react with an appropriate substrate, preferably a chromogenic substrate, in such a manner as to produce a chemical moiety that can be detected, for example, by spectrophotometric, fluorimetric or by visual means. Enzymes that can be used to detectably label the antibody include, but are not limited to, malate dehydrogenase, staphylococcal nuclease, delta-5-steroid isomerase, yeast alcohol dehydrogenase, .alpha.-glycerophosphate, dehydrogenase, triose phosphate isomerase, horseradish peroxidase, alkaline phosphatase, asparaginase, glucose oxidase, .beta.-galactosidase, ribonuclease, urease, catalase, glucose-6-phosphate dehydrogenase, glucoamylase and acetylcholinesterase. The detection can be accomplished by colorimetric methods that employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible peptides through the use of a radioimmunoassay (RIA). See, e.g., Weintraub, B., Principles of Radioimmunoassays, Seventh Training Course on Radioligand Assay Techniques, The Endocrine Society, March, 1986. The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine. The antibody can also be detectably labeled using fluorescence emitting metals such as those in the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labelling are luciferin, luciforase and acquorin.

The present description also relates to preventing and/or treating inflammatory bowel conditions in a subject in need of, comprising administering lantibiotics, as well as other antibacterial compounds, which are produced by bacteria in the digestive tract of normal individuals. A probiotic approach can be used, where bacteria that produce these compounds are administered, instead of providing the compounds in purified forms.

As described in detail above, the microbial community of subjects with inflammatory bowel conditions is perturbed. These perturbations can have profound consequences on the health of the subject. Certain bacteria, such as *Ruminococcus sp.* produce lantibiotics that have protective and antibacterial effects on pathogenic bacteria. For example, it is shown above in Table 5 above that *R. gnavus* is reduced in subjects having Crohn's disease and ulcerative colitis. *R. gnavus* produces a lantibiotic (RumA) that is active against pathogenic bacteria. The reduction in the *R. gnavus* community in these subjects can result in the growth of deleterious bacteria (such as pathogenic bacteria) that in turn is associated with an inflammatory response. Conversely, certain bacteria associated with these inflammatory bowel conditions can produce lantibiotics that inhibit beneficial bacteria such as Lactobacillus species. By providing the lantibiotic (either in purified or as a probiotic), subjects with these conditions can be treated. Any lantibiotic produced by a bacteria described herein can be utilized to prevent and/or treat inflammatory bowel conditions. The RDP group, the representative genus, or the species of the bacteria listed in Tables 3 and 5 can be utilized for diagnostic, prognostic, and disease monitoring purposes in accordance with the present invention. For instance, an increase in a Moraxella osloensis was observed in Crohns mucosa in comparison to control mucosa. This information was obtained from a sequenced clone originating in the mucosa of a Crohns patient. Sequence searching of the RDP database Version 8.1 (Cole JR, Chai B, Marsh TL, Farris RJ, Wang Q, Kulam SA, Chandra S, McGarrell DM, Schmidt TM, Garrity GM, Tiedje JM. The Ribosomal Database Project (RDP-II): previewing a new autoaligner that allows regular updates and the new prokaryotic taxonomy. Nucleic Acids Res 2003 Jan 1;31(1):442-3) (see, e.g., world wide web at rdp8.cme.msu.edu/html) indicated that it was a member of the Pseudomonas_and_relatives RDP group, and more precise sequence analysis assigned it to the Moraxella genus. For the purposes of the present invention, the RDP group alone can be used as the indicator of disease status. Thus, in the above-mentioned example, classifying a bacteria as a member of the Pseudomonas_and_relatives RDP group (irrespective of its genus or species classification) is sufficient to indicate that the patient harboring the bacteria in their intestinal mucosa is more likely to be afflicted with Crohns disease, or to be regressing from a temporary remission. One or more groups can be used diagnostically. Therefore, with respect to the example above, determining that a patient's microbial community comprises both Pseudomonas_and_relatives and Acidovorax_Group bacteria indicates the existence of Crohns disease. Similar analysis can be made for all the RDP groups disclosed in Tables 3 and 5. Although not all permutations may be disclosed in the application, they can be routinely chosen from Tables 3, 5, and the appended claims.

For any given clone isolated from a subject suspected of having Crohns, Ulcerative colitis, or Pouchitis, a SSU rRNA sequence 97% identity to a known species is generally sufficient for it to be classified as that species. Similarly, about 95% identity is generally sufficient for genus and RDP group classification. Identity was determined using the BLAST algorithm (Tatusova, T. A., & Madden, T. L. (1999). BLAST 2 Sequences, a New Tool for Comparing Protein and Nucleotide Sequences. FEMS Microbiology Letters, 174, 247-250; Altschul, S. F., Madden, T. L., Schaffer, A. A., Zhang, J., Zhang, Z., Miller, W., et al. (1997). Gapped BLAST and PSI-BLAST: a new generation of protein database search programs. Nucleic acids research, 1997 Sep 1, 25(17):3389-402; Wheeler, D. L., Chappey, C., Lash, A. E., Leipe, D. D., Madden, T. L., Schuler, G. D., et al. (2000). Database resources of the National Center for Biotechnology Information. Nucleic Acids Res, 28(1), 10-14.).

The present description provides methods diagnosing, prognosticating, and/or monitoring disease progression of Crohn's disease ulcerative colitis, or pouchitis, or in a subject, comprising: contacting a colonic mucosal tissue sample comprising nucleic acid with a polynucleotide probe which is specific for at least one bacteria under conditions effective for said probe to hybridize specifically with said nucleic acid, and detecting hybridization between said probe and said nucleic acid, wherein an increase, as compared to a normal mucosa sample, of one or more bacteria species or RDP group selected from the following indicates the disease presence or the disease status: a) Crohn's disease: *Morexella sp.* of Pseudomonas group; *Comamonas sp.* of the Acidovorax_Group; or *Cryseobacterium sp.* of the Cytophaga_Group_I (where the RDP group and/or genus and/or genus_species can be used); b) ulcerative colitis: *Morexella sp.* of Pseudomonas_and_Relatives; *Comamonas sp.* of the Acidovorax_Group; *Clostridium sp.* of the Clostridium botulinum_Group; or *Enterococus sp.* of the Enterococcus_Group (where the RDP group and/or genus and/or genus_species can be used); or c) pouchitis (compared to normal pouch): *Ruminococus sp.* of Clostridium_Coccoides_Group; *Escherichia coli* and *Shigella sp.* of the Enterics and Relatives group; or *Fusobacterium sp.* of the Fusobacteria_Group (where the RDP group and/or genus and/or genus_species can be used).

The present description also provides methods for diagnosing, prognosticating, and/or monitoring disease progression of Crohn's disease, ulcerative colitis, or pouchitis, comprising: contacting a colonic mucosal tissue sample comprising nucleic acid with a polynucleotide probe which is specific for at least one bacteria under conditions effective for said probe to hybridize specifically with said nucleic acid, and detecting hybridization between said probe and said nucleic acid, wherein a decrease, as compared to a normal mucosa sample, of one or more bacteria selected from the following group said bacteria indicates the disease presence or the disease status: a) Crohn's disease: *Bacteroides sp.* of the Bacteroides Group; *Propionibacterium sp.* of the Propionibacterium Group; or *Ruminoccocus sp.* of the Clostridium_Coccoides Group (where the RDP group and/or genus and/or genus_species can be used); b) ulcerative colitis: *Bacteroides sp.* of the Bacteroides Group; *Propionibacterium sp.* of the Propionibacterium Group; or *Ruminoccocus sp.* of the Clostridium_Coccoides Group (where the RDP group and/or genus and/or genus_species can be used); c) pouchitis: *Bacteroides sp.* of the Bacteroides Group; *Propionibacterium sp.* of the Propionibacterium Group (where the RDP group and/or genus and/or genus_species can be used).

The present description also provides methods for diagnosing, prognosticating, and/or monitoring disease progression of Crohn's disease ulcerative colitis, or pouchitis, or in a subject, comprising: determining the presence of one or more of the following bacteria in a colonic mucosal tissue from a subject having Crohn's disease, ulcerative colitis, or pouchitis: a) Crohn's disease: *Morexella sp.* of Pseudomonas group; *Comamonas sp.* of the Acidovorax_Group; or *Cryseabacterium sp.* of the Cytophaga_Group_I (where the RDP group and/or genus and/or genus_species can be used); b) ulcerative eolitis: *Morexella sp.* of Pseudomonas_and_Relatives; *Comamonas* sp. of the Acidovorax-Group; *Clostridium sp.* of the Clostridium botulinum_Group; or *Enterococus sp.* of the Enterococcus_Group (where the RDP group and/or genus and/or genus_species can be used); c) pouchitis (compared to normal pouch): Ruminococus sp. of Clostridium_Coccoides_Group; *Escherichia coli and Shigella sp.* of the Enterics and Relatives group; or *Fusobacterium sp.* of the Fusobacteria_Group (where the RDP group and/or genus and/or genus_species can be used).

The present description also provides methods for diagnosing, prognosticating, and/or monitoring disease progression of Crohn's disease, ulcerative colitis, or pouchitis, comprising: determining the absence of one or more of the following bacteria in a colonic mucosal tissue from a subject having Crohn's disease, ulcerative colitis, or pouchitis: a) Crohn's disease: *Bacteroides sp.* of the Bacteroides Group; *Propionibacterium sp.* of the Propionibacterium Group; or *Ruminoccocus sp.* of the Clostridium_Coccoides Group (where the RDP group and/or genus and/or genus_species can be used); b) ulcerative colitis: *Bacteroides sp.* of the Bacteroides Group; *Propionibacterium sp.* of the Propionibacterium Group; or *Ruminoccocus sp.* of the Clostridium-Coccoides Group (where the RDP group and/or genus and/or genus_species can be used); c) pouchitis: *Bacteroides sp.* of the Bacteroides Group; or *Propionibacterium sp.* of the Propionibacterium Group (where the RDP group and/or genus and/or genus_species can be used).

The present description also provides method for diagnosing, prognosticating, and/or monitoring disease progression of Crohn's disease or Ulcerative colitis, in a subject, comprising: contacting a lumen sample comprising nucleic acid with a polynucleotide probe which is specific for at least one bacteria under conditions effective for said probe to hybridize specifically with said nucleic acid, and detecting hybridization between said probe and said nucleic acid, wherein an increase, as compared to a normal lumen sample, of one or more bacteria selected from the following indicates the disease presence or the disease status: a) Crohn's disease: *Bacteriodes sp.* of the Bacteriodes_Group; or *Chryseobacterium sp.* of the Cytophaga_Group_I (where the RDP group and/or genus and/or genus_species can be used); or b) ulcerative colitis: *Bacteriodes sp.* of the Bacteriodes_Group; or *Chryseobacterium sp.* of the Cytophaga_Group_I (where the RDP group and/or genus and/or genus_species can be used).

The present description also provides methods for diagnosing, prognosticating, and/or monitoring disease progression of Crohn's disease or ulcerative colitis in a subject, comprising: contacting a lumen sample comprising nucleic acid with a polynucleotide probe which is specific for at least one bacteria under conditions effective for said probe to hybridize specifically with said nucleic acid, and detecting hybridization between said probe and said nucleic acid, wherein a decrease, as compared to a normal lumen sample, of *Acinetobacter sp.* or *Moraxella sp.* of the Pseudomonas and relatives group indicates that said subject has Crohn's disease or ulcerative colitis (where the RDP group and/or genus and/or genus_species can be used).

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

### EXAMPLES

Sample Collection and DNA extraction: Endoscopic mucosal tissue samples were collected from the terminal ileum, cecum+ascending colon, transverse colon, sigmoid colon and the rectum of patients with IBD and Pouchitis as well as healthy controls undergoing the colonoscopy. Some of the tissue samples were washed in saline prior to analysis to remove non-adherent bacteria (washed vs. unwashed samples). Retained lumen samples were also collected via the endoscope at the time of procedure. The samples were fingerprinted for bacterial patterns in 4 control, 2 UC, 4 CD and 3 patients with pouchitis, and 5 patients with pouch without pouchitis using the ALH methodology. The DNA extractions were performed using the Bio101 soil kit from Qbiogene, Inc, Montreal, Quebec according to the manufacturers instructions. These ALH amplicons were pooled, then cloned and sequenced to identify the bacterial components that were indicative of the disease state.

Amplicon Length Heterogeneity (ALH) Fingerpinting: ALH is a technique of bacterial fingerprinting see Ritchie, N.J., et al., Use of Length Heterogeneity PCR and Fatty Acid Methyl Ester Profiles to Characterize Microbial Communities in Soil. Applied and Environmental Microbiology, 2000. 66(4): p. 1668-1675; Suzuki, M., M.S. Rappe, and S.J. Giovannoni, Kinetic bias in estimates of coastal picoplankton community structure obtained by measurements of small-subunit rRNA gene PCR amplicon length heterogeneity. Applied and Environmental Microbiology [Appl. Environ. Microbiol.]. ALH is a PCR-based analysis which can distinguish different organisms based on natural variations in the length of 16S ribosomal DNA sequences. Purified DNA (10ng) was amplified with PCR by using a fluorescently- labeled forward primer 27F (5'-[6FAM] AGAGTTTGATCCTGGCTCA G-3') and unlabeled reverse primer 338R' (5'-GCTGCCTCCCGTAGGAGT-3'). Both primers are highly specific for eubacteria. Alternatively, we have primers that amplify the corresponding region in Archae see Burggraf, S., T. Mayer, R. Amann, S. Schadhauser, C.R. Woese and K.O. Stetter, Identifying Members of the Domain Archaea with rRNA-Targeted Oligonieleotide Probes. App. Environ. Microbiol., 1994. 60: p. 3112-3119.. We have recently optimized primers specific to the ITS of fungi and demonstrated that these region of the rRNA operon is more informative that the SSU rRNA see Borneman, J. and J. Hartin, PCR primers that amplify fungal rRNA genes from Environmental Samples. App. Environ. Microbiol., 2000. 66(10): p. 4356-4360.. The reactions were performed using 50-ul (final volume) mixtures containing 1 X PCR buffer, 0.6% bovine serum albumin, 1.5 mM MgCl2, each deoxynucleoside triphosphate at a concentration of 0.2mM, each primer at a concentration of 0.2 uM, and 2 U of *Taq* DNA polymerase. Initial denaturation at 94 C for 3 min was followed by 25 cycles consisting of denaturation at 94 C for 45 sec, annealing at 55 C for 45 s, and extension at 72 C for 2 min. There was a final extension step that consists of 72 C for 7 min. ALH samples are were stored at -20 C in the dark until used (usually less than 1 week).

The ALH PCR products were separated on the SCE9610 capillary fluorescent sequencer (Spectrumedix LLC, State College, PA) and analyzed with their GenoSpectrum software package. The software converts fluorescence data into electropherograms. The peaks of the electrophero grams represent different populations of microflora of different sizes. All fingerprinting data was analyzed using software (Interleave 1.0, BioSpherex LLC) that combines data from several runs, interleaves the various profiles, normalizes the data, and calculates diversity indices. The normalized peak areas were calculated by dividing an individual peak area by the total peak area in that profile.

Analysis of ALH Fingerprints: The diversity of ALH fingerprints were analyzed using indices of Richness (R), Evenness (E) and the Shamnon-Weaver diversity index (SW) in groups comparing IBD to controls, see Hughes, j.B., et al., Counting the Uncountable: Statistical Approaches to Estimating Microbial Diversity. App. Environ. Microbiol., 2001. 67(10): p. 4399-4406.. IBD related parameters such as disease activity, and histologically involved and uninvolved parts of the ileum & colon were compared using the diversity indices.

These fingerprints were analyzed to determine global clustering of ALH fingerprints into presence or absence of IBD, IBD types (CD, UC and pouchitis), disease activity, and involved and uninvolved parts of the ileum & colon (tissue state). Multidimensional Reductions Analysis [Principal Component Analysis (PCA), Principal Coordinate Analysis (PCO), Canonical Correspondence Analysis (CCA)] and Clustering Analysis was done using the Multi Variate Statistical Package (MVSP), Kovach Computing Services, Wales, UK. The following analyses will be done. Generation of dendograms by Unweighted Pair Group Method using Arithmetic Averages (UPGMA).

*Principal Component Analysis (PCA):* PCA is one of the best known and earliest ordination methods, first described by Karl Pearson (1901). Graphically, it is a rotation of a swarm of data points in multidimensional space so that the longest axis (the axis with the greatest variance) is the first PCA axis, the second longest axis perpendicular to the first is the second PCA axis, and so forth. The first few PCA axes represent the greatest amount of variation in the data set. The first two or three axes are generally expected to account for a large proportion of the variance, e.g. 50-60% or more.

*Principal Coordinates Analysis (PCO):* PCO can be viewed as a more general form of PCA. PCO can use a variety of different measures of distance or similarity. In general, the distances or similarities are measured between the cases directly, rather than the variables as in PCA. The main advantage of PCO is that many different kinds of similarity or distance measures can be used. PCO is restricted to analyzing distances or similarities that are metric and the distances used must be able to be viewed in some sensible geometrical manner e.g. a triangle.

*Canonical Corespondance Ana*/*ysis (CCA):* In PCA & PCO, the data are subjected to some type of mathematical manipulation in order to reveal the most important trends. These trends are then often compared to other data relating to the same samples to determine the relationship between the two. However, in CCA, the data are directly related. CCA is a multivariate direct gradient analysis method that has become very widely used in ecology.

Cluster Analysis: Cluster analysis is a term used to describe a set of numerical techniques in which the main purpose is to divide the objects of study into discrete groups. These groups are based on the characteristics of the objects and it is hoped the clusters will have some sort of significance related to the research questions being asked. Cluster analysis is used in many scientific disciplines and a wide variety of techniques have been developed to suit different types of approaches. The most commonly used ones are the agglomerative hierarchical methods. Hierarchical methods arrange the clusters into a hierarchy so that the relationships between the different groups are apparent and the results are presented in a tree-like diagram called a dendrogram. The agglomerative methods used to create a dendrogram start by successively combining the most similar objects until all are in a single, hierarchical group. Similarly dendograms can be created using the well established Unweighted Pair Group Method using Arithmetic Averages (UPGMA) and K-means.

Putative ALH fingerprint patterns (i.e. presence or absence of certain amplicon peaks) associated with IBD presence, disease types, disease activity, and tissue state were identified. For this purpose, we will visually inspect histograms of ALH fingerprints. To determine statistical correlations of peaks to IBD related variables, we also used multivariate analysis for large variable sets i.e. discriminate analysis and Canonical correspondence analysis. We also employed computerized data mining tools with supervised and unsupervised pattern recognition algorithms. These include C4.5, support vector machines, and self organizing maps. Hence, these analyses will be used to determine if ALH fingerprinting can distinguish between IBD related parameters (disease presence, type, activity, tissue state) and determine particular ALH patterns (presence or absence of a peak or sets of peaks) associated with IBD.

Sequencing of ALH Clones: The PCR product generated with primers used for ALH fingerprinting were cloned by using pGEM-T Easy Vector System II (Promega Corp., Madison, WI). Clones were screened assessing for inserts using alpha-complementation with X-Gal (5 bromo-4chloro-3indoyl-B-D-galactopyranoside) and IPTG (isopropyl-B-D-thiogalactopyranoside). Inserts were sequenced by using *Taq* dye terminator chemistry and the sequencing products were separated on a SCE9610 fluorescent capillary sequencer.

Analysis of ALH clone data: The above ALH clone sequences were compared to sequences in the RDP database to assess for patterns of microflora using a novel program (CloneID 1.0, BioSpherex LLC). The algorithm basically uses Megablast to compare the clone sequence data to the RDP database and compiles a table using the RDP numbers to correlate the identification with a hierarchical classification scheme. These same ALH clones were fingerprinted to determine the empirical ALH size and correlated with the original ALH fingerprint of sample using a second program (CloneMatch 1.0, BioSpherex, LLC).

### Results of Crohns (CD) and Ulcerative Colitis (UC) Analysis

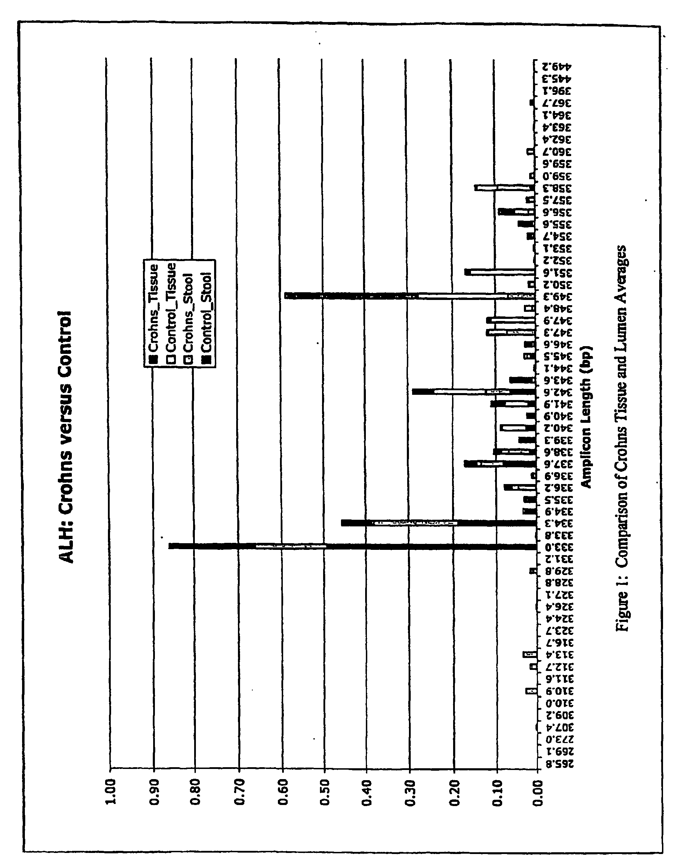

Although ALH fingerprints vary qualitatively and quantitatively between individuals, there are very distinct diagnostic patterns that can be seen from the analysis of pooled tissue (mucosa) and lumen samples. Figure 1 is a histogram compiled from the average of the ALH fingerprint from all the Crohns samples and Control samples (i.e. all individuals and all locations) showing amplicon lengths in base pairs (bp) on the x-axis and relative abundances on the y-axis. The pooled Controls Tissue samples (white bars) had very distinct ALH profile that differed dramatically from the Controls Lumen samples (black bars) indicating that there is a distinct microflora community adhering to the mucosa as a biofilm. In contrast there was not a clear differentiation between the lumen and tissue microflora in Crohns disease indicating a dramatic dysbiosis in which many of the bacterial species normally in lumen are found in the biofilm. Thus, there was much more overlap between the ALH amplicons of Crohns Tissue (light grey bars) and Crohns Lumen (dark grey bars) with Control Lumen (black bars). There are diagnostic ALH amplicons that occur predominantly in the Control Lumen samples and Crohns tissue (i.e. at 333.0 bp, 334.3 bp, and 338.6). Furthermore, there are some ALH amplicons that are unique for Crohns tissue (i.e. 310.9 bp and 313.4 bp) but on average they make up a small proportion of the microflora community.

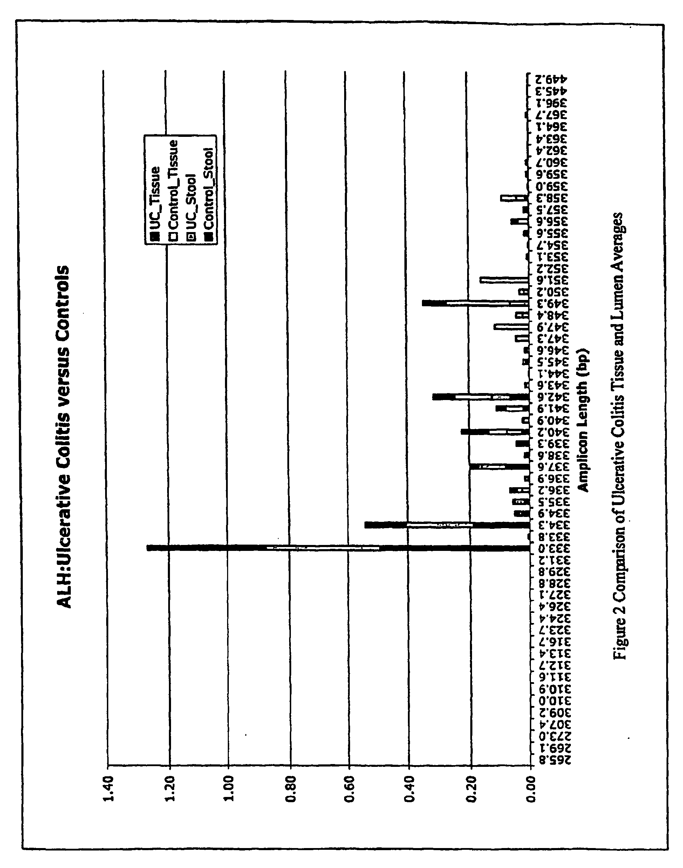

Similarly, there seems to be dysbiosis in Ulcerative colitis (UC) as the ALH profiles of UC Tissue and UC Lumen are similar to Control Lumen with the ALH profile of the Controls being very distinct (Figure 2). Thus, there was much more overlap between the ALH amplicons of UC Tissue (light grey bars) and UC Lumen (dark grey bars) with Control Lumen (black bars). Some of the diagnostic ALH amplicons that were observed in CD (see above) are the same amplicons that are diagnostic in UC (i.e. at 333.0 bp, 334.3 bp, and 338.6 bp). Furthermore, there are distinct ALH amplicons that occur only in the UC tissue (i.e. 334.6 bp).

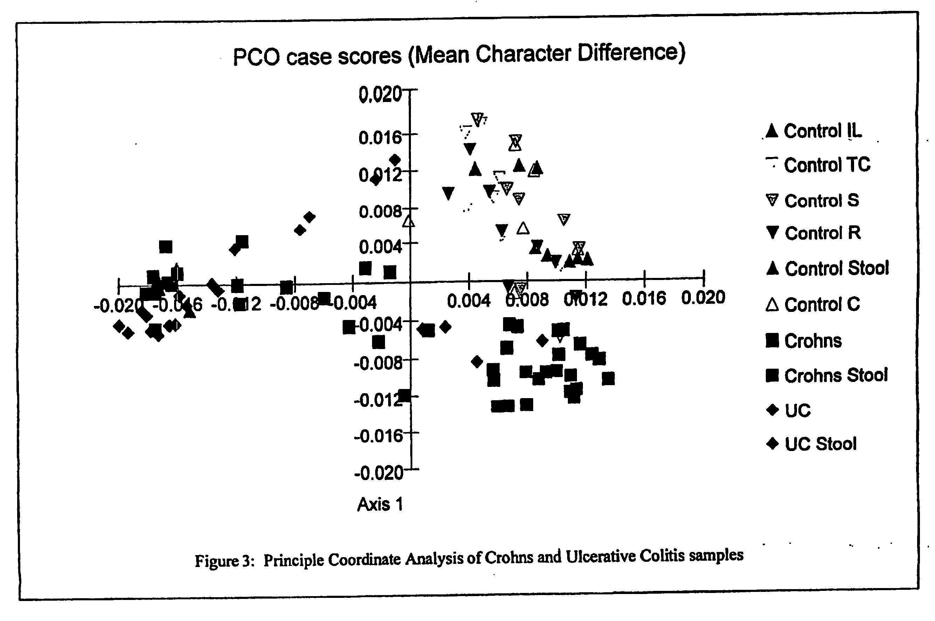

When the mean character differences for ALH profiles from Controls, CD, and UC were examined using Principle Coordinate Analysis (PCO), dramatic clustering patterns can be seen for UC and CD that is distinct from the Control samples (Figure 3). We clearly see distinct clustering of Control ALH profiles in the 1^{st} quadrant, UC clusters in the 3^{rd} & 4^{th} quadrants boundary, and CD is mainly clustered in the 2^{nd} quadrant. It is also important to note that the lumen samples cluster in the 3^{rd} & 4^{th} quadrant associated with UC. There are also several Crohns ALH profiles that cluster in this 3^{rd} & 4^{th} quadrants suggesting that there is variation in the tissue microflora of Crohns and that, in specific samples, these ALH profiles are similar to those of UC.

PCA and Canonical Correspondence Analysis demonstrates a similar clustering of healthy controls separate from CD and UC patients. The dendograms produced with UPGMA clustering using a Jacard distance measure also show the same general patterns as the PCO analysis.

We have cloned and sequenced pooled ALH amplicons from the UC, CD and healthy controls samples and these sequence data were used to identify the bacterial species associated with each disease state. Table 1 summarizes the key bacterial groups based on the RDP classification scheme that occur at a frequency of greater than 5% of the microfloral community. The data supports the ALH profiles in that the microflora found on the mucosal surface of CD and UC tissue resemble the microfloral composition of lumen in healthy individuals and that this composition differs from the microfloral composition of the controls mucosa. Specifically, members of the Pseudomonads such as *Moraxella sp*. and members of the Acidovorax group such as *Comoamonas sp*. are associated with Control lumen, CD lumen, CD mucosa, UC lumen, and UC mucosa. Additionally, members of the Cyotphaga group such as Chryseobacterium balustinum are associated with CD mucosa, CD lumen, and UC lumen. Finally, members of both of the Clostridium group (*Clostridium paraputrificum*) and Enterococcus (*Enterococcus hirae*) are also associated with UC mucosa. We also note that there is a quantitative decrease in the Bacteroides group in UC mucosa and CD mucosa compared to the Control mucosa. In summary, we conclude that there are bacterial species that are associated in the CD biofilm and UC biofilm that are normally found in lumen and that this indicates severe dysbiosis.

### Results for Pouchitis Analysis

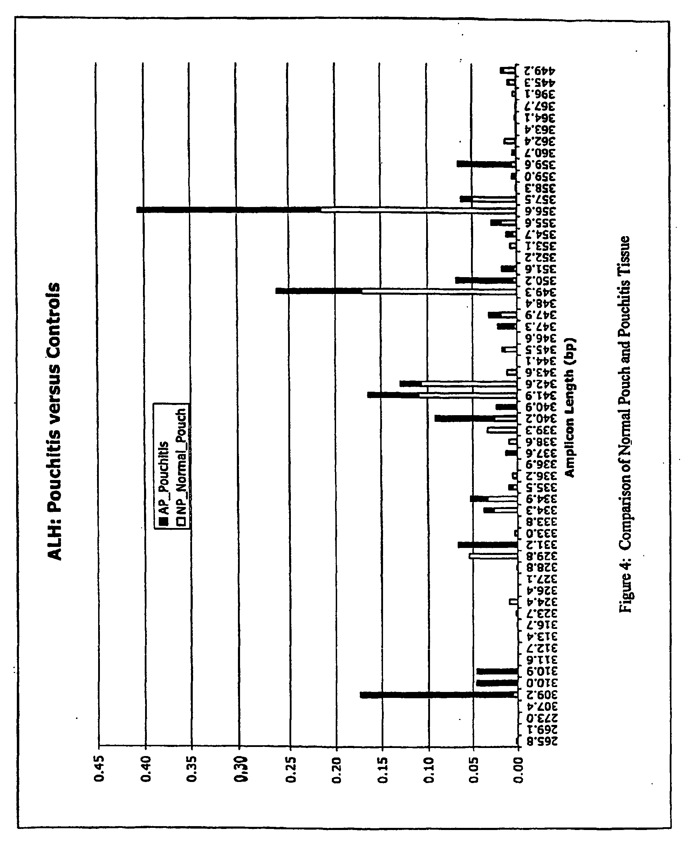

Figure 4 is a histogram compiled from the average of the ALH fingerprint from all the Pouchitis samples (AP) and Normal pouch samples (NP), that is samples from patients with active Pouchitis (AP) and patients with a Pouch but are normal upon examination (NP). As seen in CD and UC, the pooled NP mucosa samples (white bars) had very distinct ALH profile that differed dramatically from the NP mucosa samples (black bars) indicating that there is a distinct microflora community adhering to the mucosa as a biofilm. Furthermore, the ALH amplicon profiles from the NP samples were different that healthy control patients that did not have a Pouch. There are diagnostic ALH amplicons that occur predominantly in the AP mucosa samples (i.e. at 309.2 bp, 310.0 bp, 310.9 bp, 331.2 bp, 350.2 bp, and 359.6 bp) that are not the predominant diagnostic ALH amplicons in CD and UC. Thus, the dysbiosis in Pouchitis seems to be very different from CD and UC and may involve different pathology. Furthermore, the actual components of the community in the disease state vary from individual to individual. For example, the ALH amplicons at 309.2 bp, 310.0 bp, 310.9 bp are major components in one patient but are only minor components of others. Importantly, the Normal Pouch patients have abnormal microflora content in the mucosal biofilm and these patients may be continuously in a semi-disease state.

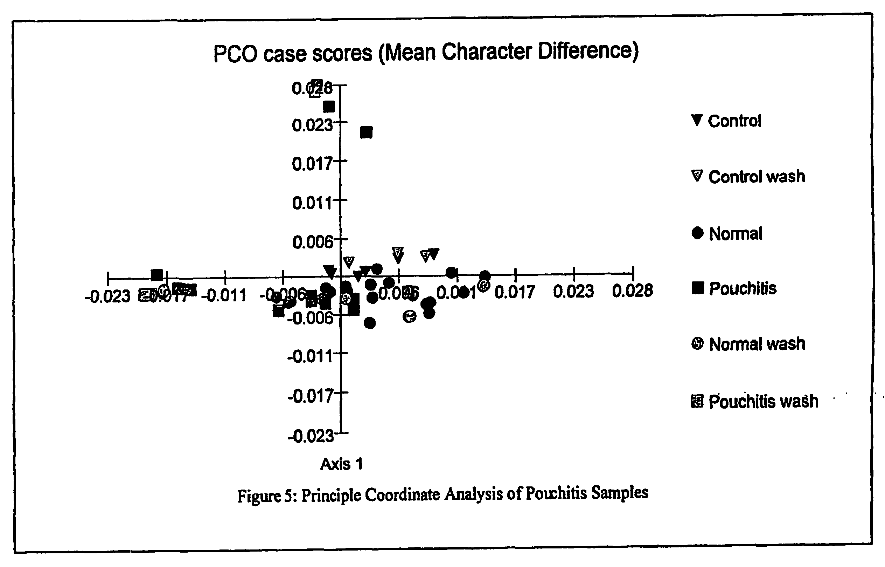

When the mean character differences for ALH profiles from NP and AP samples were examined using Principle Coordinate Analysis (PCO), a general clustering pattern can be seen for NP in the center of the graph that is distinct from three clusters of AP samples (Figure 5). Interestingly, each of these AP clusters are from separate patient confirming that patients with Pouchitis exhibit much more variation in the microflora in the mucosal biofilm. It should be noted that the separate cluster found on the Y axis above the cluster of Normal Pouch is the patient that displayed the distinct ALH amplicons at 309.2 bp, 310.0 bp, 310.9 bp. The extent of activity of the disease may be reflected in the extent of dysbiosis depicted in the PCO plot. Furthermore, the pattern is consistent whether the samples have been washed or not washed in saline as is the case in the CD and UC samples.

We have cloned and sequenced pooled ALH amplicons from the NP and AP mucosal samples and these sequence data were used to identify the bacterial species associated with each disease state. Table 1 summarizes the key bacterial groups based on the RDP classification scheme that occur at a frequency of greater than 5% of the microfloral community. The data supports the ALH profiles in that the microflora found on the mucosal surface of both AP and NP tissue are different from that found in healthy individuals and these do not reflect the microflora found in Normal lumen as found in CD and UC. Specifically, members of the Clotridium group (i.e. *Clostridium paraputrificum*), members of Enterics (i.e. *E.coli and Shigella sp*.), and members of the Streptococcus group (i.e. *Streptococcus brevis*) are found associated with the mucosa of NP patients. On the other hand, the microflora associated with the mucosa in AP patients was very diverse and differed from the NP patients. Specifically, we observed that members of the Enterics (i.e. *E.coli and Shigella sp.*) and Fusobacterium group (i.e. *Fusobacterium varium*) was associated with the mucosa in the AP patients and that there was a dramatic loss of members of the Streptococci group (i.e. *Streptococcus brevis*). Furthermore, a different *Ruminococcus* species (*Ruminococcus obeum)* was associated with AP patients but it is not clear that this strain difference would contribute to the pathology. In summary, it looks like both NP and AP patients have dysbiosis compared to the normal controls and that there is a dramatic loss of Streptococci in AP patients. Furthermore, there seems to be patient specific (see Figure 5) ALH fingerprints suggesting significant variation in the microflora between patients.

### Correlation of ALH Amplicons and Microflora

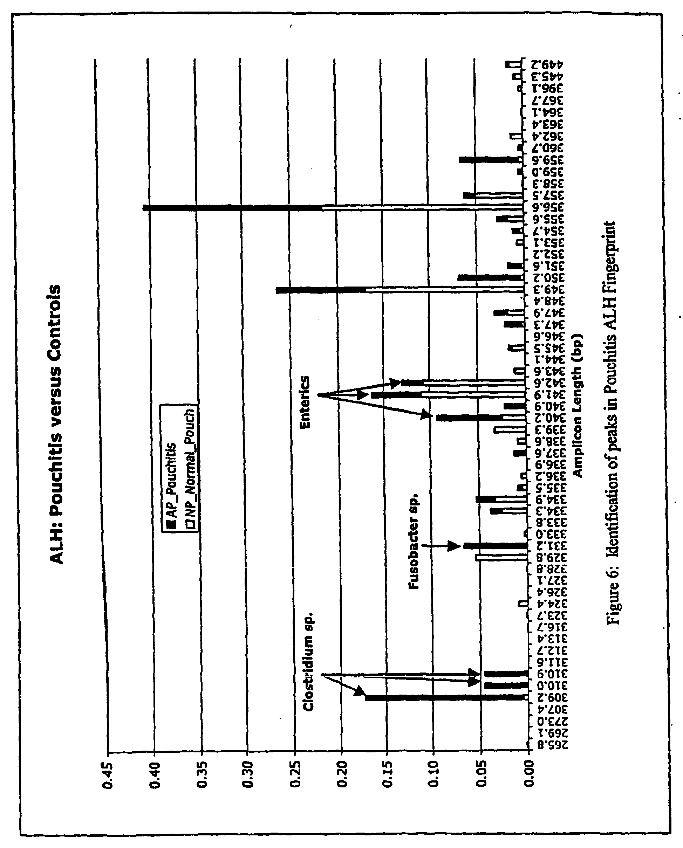

We have correlated the experimentally determinedALH amplicon size of clones with the identifications obtained from sequencing these. For example, we have labeled the main amplicons in the histogram Pouchitis and Normal Pouch ALH fingerprints in Figure 6. We then use this information to correlate what bacterial species are in the diagnostic peaks of the ALH profiles.

The preceding examples can be repeated with similar success by substituting the generically or specifically described reactants and/or operating conditions of this invention for those used in the preceding examples.

**Table 1 Increase or Decrease of ALH Fingerprint amplicons greater than 5% in Crohns mucosa versus Control Mucosa**

| Amplicon Size (bp) | 333.0 | 334.3 | 336.2 | 337.6 | 338.6 | 340.2 | 341.9 | 342.6 | 343.6 | 347.3 | 347.9 | 349.3 | 351.6 | 358.3 |
|---|---|---|---|---|---|---|---|---|---|---|---|---|---|---|
| Control Mucosa | | | 2% | 1% | | 6% | 6% | 12% | 1% | 4% | 11% | 21% | 16% | 5% |
| Control Mucosa | 20% | 7% | 2% | 3% | 2% | | 3% | 5% | 6% | | 1% | 31% | 1% | |
| Increased in Crohns mucosa | 333.0 | 334.3 | | | 338.6 | | | | 343.6 | | | | | |
| Decreased in Crohns mucosa | | | | | | 340.2 | 341.9 | 342.6 | | 347.9 | 347.9 | 349.3 | 351.6 | 358.3 |

**Table 2 Increase or Decrease of ALH Fingerprint amplicons greater than 5% in Ulcerative Colitis mucosa versus Control Mucosa**

| Amplicon Size (bp) | 333.0 | 334.3 | 340.2 | 341.9 | 342.6 | 347.9 | 349.3 | 351.6 | 358.3 |
|---|---|---|---|---|---|---|---|---|---|
| Control Mucosa | | | 6% | 6% | 12% | 11% | 21 % | 16% | 5% |
| UC Mucosa | 39% | 13% | 9% | 3% | 7% | | 7% | | |
| Increased in UC mucosa | 333.0 | 334.3 | 340.2 | | | | | | |
| Decreased in UC mucosa | | | | 341.9 | 342.6 | 347.9 | 349.3 | 351.6 | 358.3 |

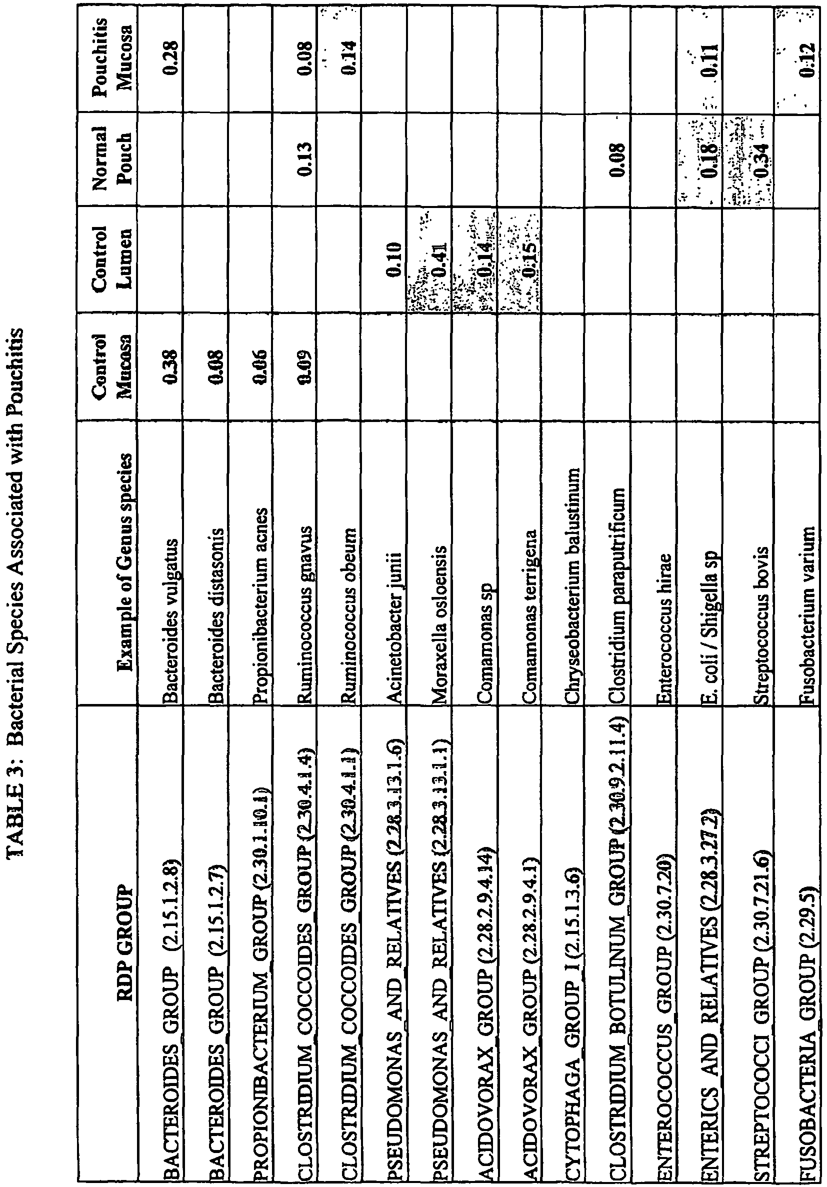

**Table 4 Increase or Decrease of ALH Fingerprint amplicons greater than 5% in Pouchitis mucosa versus Normal Pouch**

| Amplicon Size (bp) | 309.2 | 310.0 | 310.9 | 329.8 | 331.2 | 340.2 | 341.9 | 342.6 | 349.3 | 350.2 | 356.6 | 357.5 | 359.6 |
|---|---|---|---|---|---|---|---|---|---|---|---|---|---|
| Normal Pouch | 1% | | | 5% | | 2% | 11% | 11% | 17% | | 32% | 5% | 1% |
| Pouchitis | 17% | 5% | 5% | | 7% | 7% | 5% | 2% | 9% | 6% | 19% | 1% | 6% |
| Increased in Pouchitis | 309.2 | 310.0 | 310.9 | | 331.2 | 340.2 | | | | 350.2 | 356.6 | | 359.6 |
| Decreased in Pouchitis | | | | 329.8 | | | 341.9 | 342.6 | 349.3 | | | 357.5 | |

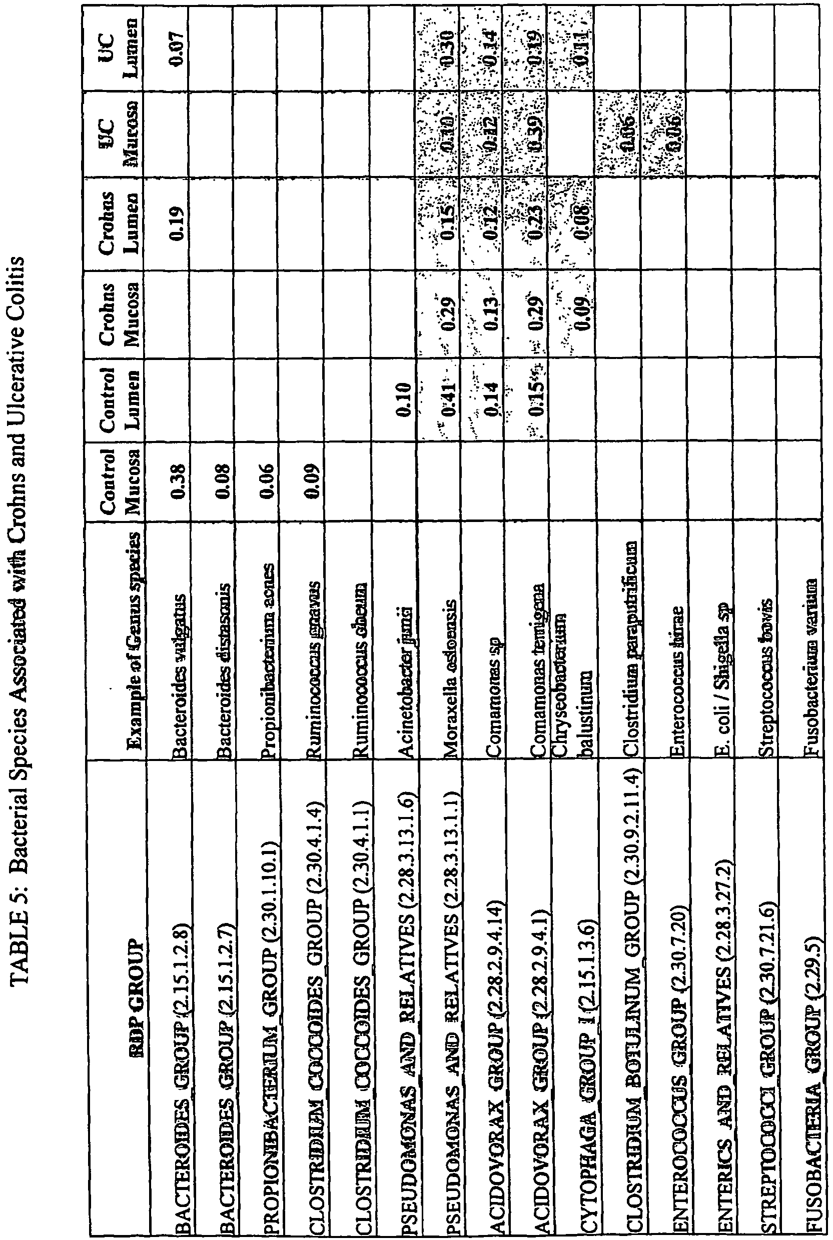

**TABLE 6**

| **RDP REFERENCE** | **SEQ ID NO** | **GENUS SPECIES** |
|---|---|---|
| 2.28-3.13.1.6 | 14 | Acinetobacter junii |
| 2.15.1.2.7 | 1 | Bacteroides distasonis |
| 2.15.1.2.8 | 2 | Bacteroides fragilis |
| 2.15.1.2.8 | 3 | Bacteroides ovatus |
| 2.15.1.2.8 | 4 | Bacteroides vulgatus |
| 2.15.1.3.6 | 17 | Bergeyella zoohelcum |
| 2.15.1.3.6 | 18 | Chryseobacterium balustinum str. SBR1044 |
| 2.15.1.3.6 | 19 | Chryseobacterium balustinum str. SBR2024 |
| 2.28.2.9.4.1 | 16 | Comamonas terrigena |
| 2.28.2.9.4.14 | 15 | Comamonas sp. |
| 2.28.3.13.1.1 | 11 | Moraxella cuniculi |
| 2.28.3.13.1.1 | 12 | Moraxella lacunata |
| 2.28.3.13.1.1 | 13 | Moraxella osloensis |
| 2.28.3.27.2 | 25 | Escherichia coli |
| 2.28.3.27.2 | 26 | Salmonella bovis |
| 2.28.3.27.2 | 27 | Shigella boydii |
| 2.28.3.27.2 | 28 | Shigella dysenteriae |
| 2.28.3.27.2 | 29 | Shigella flexneri |
| 2.29.5 | 34 | Fusobacterium alocis |
| 2.29.5 | 35 | Fusobacterium nucleatum |
| 2.29.5 | 36 | Fusobacterium varium |
| 2.30.1.10.1 | 5 | Propionibacterium acnes |
| 2.30.4.1.1 | 10 | Clostridium sp. |
| 2.30.4.1.4 | 6 | Clostridium nexile |
| 2.30.4.1.4 | 7 | Eubacterium formicigenerans |
| 2.30.4.1.4 | 8 | Ruminococcus gnavus |
| 2.30.4.1.4 | 9 | Ruminacoccus torques |
| 2.30.7.20 | 21 | Enterococcus cecorum |
| 2.30.7.20 | 22 | Enterococcus columbae |
| 2.30.7.20 | 23 | Enterococcus hirae |
| 2.30.7.20 | 24 | Tetragenococcus halophilus |
| 2.30.7.2.1.6 | 30 | Streptococcus bovis |
| 2.30.7.21.6 | 31 | Streptococcus infantarius |
| 2.30.7.21.6 | 32 | Streptococcus salivarius |
| 2.30.7.21.6 | 33 | Streptococcus thermophilus |
| 2.30.9.2.11.4 | 20 | Clostridium paraputrificum |

## Claims

1. A method for diagnosing an inflammatory bowel disease in a subject, comprising determining the increase or decrease compared to a normal mucosa sample of *Moraxel*/*a sp.* of the Pseudomonas group, *Comamonas sp.* of the Acidovorax Group, *Cryseobacterium sp.* of the Cytophaga Group I, *Enterococcus sp.* of the Enterococcus Group, *Bacteroides sp.* of the Bacteroides Group, *Propionibacterium sp.* of the Propionibacterium Group, *Ruminococcus sp.* of the Clostridium Coccoides Group and *Bacteroides sp.* in a colonic mucosal tissue sample from said subject, wherein
(a) the inflammatory bowel disease is Crohn's disease and wherein an increase of *Moraxella sp., Comamonos sp.* and *Chryseobacter sp.* and a decrease of *Bacteroides sp., Propionibacterium sp., Enterococcus sp.* and *Ruminococcus sp.* indicates Crohn's disease in said subject, or wherein
(b) the inflammatory bowel disease is ulcerative colitis and wherein an increase of *Moraxella sp., Comamonas sp.* and *Enterococcus sp.* and a decrease of *Bacteroides sp., Propionibacterium sp., Chryseobacter sp.* and *Ruminococcus sp.* indicates ulcerative colitis in said subject.

2. The method according to claim 1, additionally comprising determining the increase or decrease of one or more bacteria selected from the group consisting of *Bacteriodes sp., Ruminococcus sp., Moraxella sp.* and *Comamonas sp.* in a lumen sample of the subject.

3. A method for diagnosing pouchitis in an ileal-pouch patient, comprising detecting *Fusobacter sp., Propionibacterium sp.* and at least one of the bacteria of the group of *E.coli* and/or *Shigella sp.* in a colonic mucosal tissue sample from said patient, wherein an increase of *Fusobacter sp.* and a decrease of one or more of the bacteria of the group of *E.coli* and/or *Shigella sp.* as compared to a normal pouch sample and the absence of *Propionibacterium sp.* is diagnostic of pouchitis.

4. The method of any of claims 1 to 3, wherein the bacteria are detected using an antibody specific for said bacteria.

5. The method of any of claims 1 to 3, wherein the bacteria are detected using an oligonucleotide probe specific for said bacteria.

6. The method of claim 5, wherein the bacteria are detected using nucleic acid fingerprinting, PCR, nucleotide sequencing, Southern blot, and/or DNA microarrays comprising a plurality of sequences specific for one or more of said bacteria.

7. The method according to any one of the claims 5 or 6, wherein said oligonucleotide probe hybridizes to small subunit ribosomal RNA or to large subunit ribosomal RNA.

8. The method according to claim 7, wherein said oligonucleotide probe hybridizes to variable regions or helix regions of said ribosomal RNA.

9. The use of a method according to any one of the claims 1 to 8 for prognosticating and/or monitoring disease progression of an inflammatory bowel disease in a subject.

## Patentansprüche

1. Verfahren zur Diagnose einer entzündlichen Darmerkrankung in einem Individuum, umfassend die Bestimmung der Zunahme oder Abnahme von *Moraxella sp.* der Pseudomonas-Gruppe, *Comamonas sp.* der Acidovorax-Gruppe, *Cryseobacterium sp.* der Cytophaga-Gruppe I, *Enterococcus sp.* der Enterococcus-Gruppe, *Bacteroides sp.* der Bacteroides-Gruppe, *Propionibacterium sp.* der Propionibacterium-Gruppe, *Ruminococcus sp.* der Clostridium Coccoides-Gruppe und *Bacteroides sp.* in einer Probe der Dickdarmmukosa des Individuums verglichen mit einer normalen Mukosaprobe, wobei
(a) die entzündliche Darmerkrankung Morbus Crohn ist und wobei eine Zunahme von *Moraxella sp., Comamonas sp.* und *Chryseobacter sp.* und eine Abnahme von *Bacteroides sp., Propionibacterium sp., Enterococcus sp.* und *Ruminococcus sp.* Morbus Crohn in dem Individuum anzeigt, oder wobei
(b) die entzündliche Darmerkrankung Colitis ulcerosa ist und wobei eine Zunahme von *Moraxella sp., Comamonas sp.* und *Enterococcus sp.* und eine Abnahme von *Bacteroides sp., Propionibacterium sp., Chryseobacter sp.* und *Ruminococcus sp.* Colitis ulcerosa in dem Individuum anzeigt.

2. Verfahren nach Anspruch 1 zusätzlich umfassend die Bestimmung der Zunahme oder Abnahme von einem oder mehreren Bakterien ausgewählt aus der Gruppe bestehend aus *Bacteroides sp., Ruminococcus sp., Moraxella sp.* und *Comamonas sp.* in einer Lumenprobe des Individuums.

3. Verfahren zur Diagnose von Pouchitis in einem Patienten mit einem Ileum-Pouch, umfassend die Bestimmung von *Fusobacter sp., Propionibacterium sp.* und mindestens einem Bakterium der Gruppe von *E.coli* und/oder *Shigella sp.* in einer Probe der Dickdarmmukosa des Patienten, wobei eine Zunahme von *Fusobacter sp.* und eine Abnahme von einem oder mehreren Bakterien der Gruppe von *E.coli* und/oder *Shigella sp.* verglichen mit einer normalen Pouch-Probe und die Abwesenheit von *Propionibacterium sp.* diagnostisch für Pouchitis ist.

4. Verfahren nach einem der Ansprüche 1 bis 3, wobei die Bakterien unter Verwendung eines Antiköpers nachgewiesen werden, der spezifisch für die Bakterien ist.

5. Verfahren nach einem der Ansprüche 1 bis 3, wobei die Bakterien unter Verwendung einer Oligonucleotidsonde nachgewiesen werden, die spezifisch für die Bakterien ist.

6. Verfahren nach Anspruch 5, wobei die Bakterien unter Verwendung des Nucleinsäure-Fingerabdrucks, PCR, Nucleotidsequenzierung, Southern Blot und/oder DNA-Microarrays nachgewiesen werden, die eine Vielzahl von Sequenzen umfassen, die für ein oder mehrere Bakterien spezifisch sind.

7. Verfahren nach einem der Ansprüche 5 oder 6, wobei die Oligonucleotidsonde mit der kleinen Untereinheit der ribosomalen RNA oder der großen Untereinheit der ribosomalen RNA hybridisiert.

8. Verfahren nach Anspruch 7, wobei die Oligonucleotidsonde mit variablen Regionen oder Helixregionen der ribosomalen RNA hybridisiert.

9. Verwendung eines Verfahrens nach einem der Ansprüche 1 bis 8 zur Prognose und/oder Beobachtung des Krankheitsverlaufs einer entzündlichen Darmerkrankung in einem Individuum.

## Revendications

1. Procédé de diagnostic d'une maladie intestinale inflammatoire chez un sujet, comprenant la détermination de l'augmentation ou de la diminution, par rapport à un échantillon muqueux normal, de *Moraxella sp.* du groupe Pseudomonas, de *Comamonas sp.* du groupe Acidovorax, de *Cryseobacterium sp.* du groupe I des Cytophaga, *d'Enterococcus sp.* des entérocoques, de *Bacteroides sp.* du groupe des bactéroïdes, de *Propionibacterium sp.* du groupe Propionibacterium, de *Ruminococcus sp.* du groupe Clostridium Coccoides et de *Bacteroides sp.* dans un échantillon de tissu muqueux du côlon dudit sujet, dans lequel
(a) la maladie intestinale inflammatoire est la maladie de Crohn, et dans lequel une augmentation de *Moraxella sp.,* de *Comamonas sp.* et de *Chryseobacter sp.* et une diminution de *Bacteroides sp.,* de *Propionibacterium sp.,* de *Enterococcus sp.* et de *Ruminococcus sp.* indiquent une maladie de Crohn chez ledit sujet, ou dans lequel
(b) la maladie intestinale inflammatoire est la rectocolite hémorragique et dans lequel une augmentation de *Moraxella sp.,* de *Comamonas sp.* et de *Enterococcus sp* et une diminution de *Bacteroides sp.,* de *Propionibacterium sp.,* de *Chryseobacter sp.* et de *Ruminococcus sp.* indiquent une rectocolite hémorragique chez ledit sujet.

2. Procédé selon la revendication 1, comprenant en outre la détermination de l'augmentation ou de la diminution d'une ou plusieurs bactéries choisies dans le groupe comprenant *Bacteroides sp., Ruminococcus sp., Moraxella sp.,* et *Comamonas sp.* dans un échantillon luminal du sujet.

3. Procédé de diagnostic d'une pochite chez un patient ayant un réservoir iléal, comprenant la détection de *Fusobacter sp.,* de *Propionibacterium sp.* et d'au moins l'une des bactéries du groupe comprenant *E. coli* et/ou *Shigella sp.* dans un échantillon de tissu muqueux du côlon dudit patient, dans lequel une augmentation de *Fusobacter sp.* et une diminution d'une ou plusieurs des bactéries du groupe comprenant *E. coli* et/ou *Shigella sp.* par rapport à un échantillon de réservoir normal et l'absence de *Propionibacterium sp.* sont un diagnostic de pochite.

4. Procédé selon l'une quelconque des revendications 1 à 3, dans lequel les bactéries sont détectées en utilisant un anticorps spécifique de ladite bactérie.

5. Procédé selon l'une quelconque des revendications 1 à 3, dans lequel les bactéries sont détectées en utilisant une sonde d'oligonucléotides spécifique desdites bactéries.

6. Procédé selon la revendication 5, dans lequel les bactéries sont détectées en utilisant le procédé d'empreintes d'acide nucléique, la PCR, le séquençage nucléotidique, le procédé Southern blot et/ou des puces à ADN comprenant une pluralité de séquences spécifiques d'une ou plusieurs desdites bactéries.

7. Procédé selon l'une quelconque des revendications 5 ou 6, dans lequel ladite sonde d'oligonucléotides s'hybride à une petite sous-unité d'ARN ribosomique ou une grande sous-unité d'ARN ribosomique.

8. Procédé selon la revendication 7, dans lequel ladite sonde d'oligonucléotides s'hybride à des régions variables ou des régions en hélice dudit ARN ribosomique.

9. Utilisation d'un procédé selon l'une quelconque des revendications 1 à 8, pour pronostiquer et/ou surveiller l'évolution pathologique d'une maladie intestinale inflammatoire chez un sujet.
